(19) Europäisches Patentamt / European Patent Office / Office européen des brevets

(11) EP 1 406 623 B1

(12) EUROPÄISCHE PATENTSCHRIFT

(45) Veröffentlichungstag und Bekanntmachung des Hinweises auf die Patenterteilung:
**12.12.2007 Patentblatt 2007/50**

(21) Anmeldenummer: **02745424.8**

(22) Anmeldetag: **03.07.2002**

(51) Int Cl.:
***A61K 31/445*** (2006.01) ***A61K 31/4545*** (2006.01)
***C07D 211/26*** (2006.01) ***C07D 211/32*** (2006.01)
***C07D 211/70*** (2006.01) ***C07D 401/12*** (2006.01)

(86) Internationale Anmeldenummer:
**PCT/EP2002/007379**

(87) Internationale Veröffentlichungsnummer:
**WO 2003/004026 (16.01.2003 Gazette 2003/03)**

# (54) SUBSTITUIERTE 1-PHENETHYLPIPERIDINVERBINDUNGEN, DIE UNTER ANDEREM ALS ANALGETIKA VERWENDUNG FINDEN

SUBSTITUTED 1-PHENETHYLPIPERIDINE COMPOUNDS USED AS INTER ALIA ANALGESICS

COMPOSES SUBSTITUES DE 1-PHENETHYLPIPERIDINE UTILISES ENTRE AUTRES COMME ANALGESIQUES

(84) Benannte Vertragsstaaten:
**AT BE BG CH CY CZ DE DK EE ES FI FR GB GR IE IT LI LU MC NL PT SE SK TR**
Benannte Erstreckungsstaaten:
**LT LV RO SI**

(30) Priorität: **05.07.2001 DE 10132746**

(43) Veröffentlichungstag der Anmeldung:
**14.04.2004 Patentblatt 2004/16**

(73) Patentinhaber: **Grünenthal GmbH**
**52078 Aachen (DE)**

(72) Erfinder:
- **SUNDERMANN, Bernd**
  **52066 Aachen (DE)**
- **HOENEN, Lambert**
  **52074 Aachen (DE)**
- **BUSCHMANN, Helmut**
  **E-08950 Esplugues de Llobregat (ES)**
- **KÖGEL, Babette-Yvonne**
  **52379 Langerwehe-Hamich (DE)**
- **FRIDERICHS, Elmar**
  **52223 Stolberg (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:
**US-A- 4 248 877** **US-A- 5 446 052**

- **HUEGI B S ET AL: "SYNTHESIS AND PHARMACOLOGICAL STUDIES OF 4,4-DISUBSTITUTED PIPERIDINES: A NEW CLASS OF COMPOUNDS WITH POTENT ANALGESIC PROPERTIES" JOURNAL OF MEDICINAL CHEMISTRY, AMERICAN CHEMICAL SOCIETY. WASHINGTON, US, Bd. 26, Nr. 1, 1983, Seiten 42-50, XP000608931 ISSN: 0022-2623**

EP 1 406 623 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft substituierte 1-Phenethylpiperidinverbindungen, Verfahren zu deren Herstellung, Arzneimittel enthaltend diese Verbindungen sowie die Verwendung dieser Verbindungen zur Herstellung von Arznelmitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Noziception in letzter Zeit erschienen sind.

US 4248877 and Huegi B.S. et ae, J. Med. Chem, 1983, 26, 42-50 beschreiben Phenylethylpiperidine mit schmerzhemmender Wirkung.

Klassische Opioide, wie beispielsweise das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie Toleranzentwicklung auf. Nach weiteren schmerzhemmenden Mitteln wird weltwelt geforscht.

Aufgabe der vorliegenden Erfindung war es daher, neue Wirkstoffe zur Verfügung zu stellen, die sich insbesondere als pharmazeutische Wirkstoffe in Arznelmitteln eignen.

Diese Wirkstoffe sollen sich insbesondere zur Bekämpfung von Schmerz, zur Behandlung von Migräne, Diarrhoe, Harninkontinenz, Pruritus, inflammatorischen Reaktionen, allergischen Reaktionen, Alkohol- und/oder Drogen- und/oder Medikamentenabhängigkeit, Alkohol- und/oder Drogen- und/oder Medikamentenmißbrauch, Entzündungen oder zur Lokalanästhesie eignen.

Erfindungsgemäß wird diese Aufgabe durch die Bereitstellung substituierter 1-Phenethylpiperidinverbindungen der nachstehenden allgemeinen Formel I gelöst die eine ausgeprägte analgetische Wirkung aufweisen und die sich Insbesondere auch zur Behandlung von Migräne, Diarrhoe, Haminkontinenz, Pruritus, inflammatorischen Reaktionen, allergischen Reaktionen, Alkohol- und/oder Drogen- und/oder Medikamentenabhängigkeit, Alkohol- und/oder Drogen- und/oder Medikamentenmißbrauch, Entzündungen oder zur Lokalanästhesie eignen.

Ein Gegenstand der vorliegenden Erfindung sind daher substituierte 1-Phenethylpiperidinverbindungen der allgemeinen Formel I

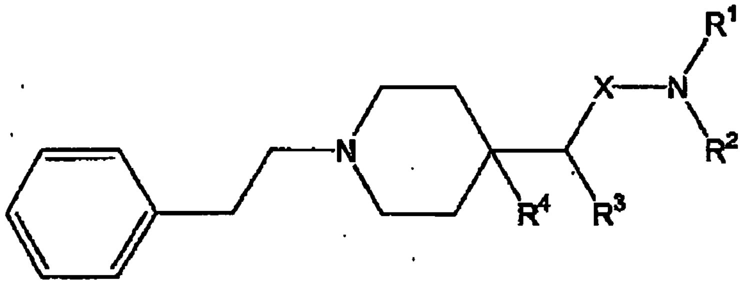

I,

worin

X für eine Methylen-($CH_2$)- oder Carbonyl-(C=O)-Gruppe, vorzugsweise für eine Methylen-($CH_2$)-Gruppe steht,

$R^1$ für einen gegebenenfalls wenigstens einfach substituierten Aryl- oder Heteroaryl-Rest, vorzugsweise für einen gegebenenfalls wenigstens einfach substituierten Aryl-Rest, steht,

$R^2$ für H, $COR^5$, $SO_2R^5$, einen gegebenenfalls wenigstens einfach substituierten, gesättigten, verzweigten oder unverzweigten aliphatischen $C_{1-10}$-Rest, einen gegebenenfalls wenigstens einfach substituierten, zumindest einfach ungesättigten, verzweigten oder unverzweigten aliphatischen $C_{2-10}$-Rest, einen gegebenenfalls wenigstens einfach substituierten, gesättigten oder zumindest einfach ungesättigten cycloaliphatischen $C_{3-8}$-Rest, einen gegebenenfalls wenigstens einfach substituierten Aryl- oder Heteroaryl-Rest oder für einen über eine $C_{1-3}$-Alkylen-Gruppe gebundenen, gegebenenfalls wenigstens einfach substituierten-Aryl- oder Heteroaryl-Rest, vorzugsweise für H, $COR^5$,

$SO_2R^5$ oder einen $C_{1-6}$-Alkyl-Rest, besonders bevorzugt für H oder $COR^5$, steht,

$R^3$ und $R^4$ jeweils für H oder zusammen für eine Bindung, vorzugsweise jeweils für H, stehen,

$R^5$ für einen gegebenenfalls wenigstens einfach substituierten, gesättigten, verzweigten oder unverzweigten aliphatischen $C_{1-10}$-Rest, einen gegebenenfalls wenigstens einfach substituierten, zumindest einfach ungesättigten, verzweigten oder unverzweigten aliphatischen $C_{2-10}$-Rest, einen gegebenenfalls wenigstens einfach substituierten, gesättigten oder zumindest einfach ungesättigten cycloaliphatischen $C_{3-8}$-Rest, einen gegebenenfalls wenigstens einfach substituierten Aryl- oder Heteroaryl-Rest oder für einen über eine $C_{1-3}$-Alkylen-Gruppe gebundenen, gegebenenfalls wenigstens einfach substituierten Aryl- oder Heteroaryl-Rest, vorzugsweise für einen $C_{1-6}$-AlkylRest oder für einen gegebenenfalls wenigstens einfach substiuierten Aryl-Rest, steht,

als freie Base oder eines entsprechenden physiologisch verträglichen Salzes sowie entsprechender Racemate, Enantiomeren und Diastereomeren.

Die aliphatischen Reste können einfach oder mehrfach substituiert sein. Sofern diese Reste mehr als einen Substituenten aufweisen, können diese gleich oder verschieden sein und sowohl an dem selben wie auch an verschiedenen Atomen des aliphatischen Restes gebunden sein. Die aliphatischen Reste können mit einem Halogen-Rest und/ oder einer Hydroxyl-Gruppe, besonders bevorzugt mit F und/oder Cl substituiert sein.

Gesättigte aliphatische Reste können bevorzugt ausgewählt werden aus der Gruppe bestehend aus gegebenenfalls wenigstens einfach substituiertem Methyl, Ethyl, Propyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl und 1-Methylpentyl. Substituierte aliphatische Reste können besonders bevorzugt $CHF_2$ oder $CF_3$ sein.

Ungesättigte aliphatische Reste können bevorzugt ausgewählt werden aus der Gruppe bestehend aus Vinyl (Ethenyl), Allyl (2-Propenyl) und 1-Propinyl.

Die cycloaliphatischen Reste können einfach oder mehrfach substituiert sein. Sofern die cycloaliphatischen Reste mehr als einen Substituenten aufweisen, können diese gleich oder verschieden sein und sowohl an dem selben wie auch an verschiedenen Atomen des cycloaliphatischen Restes gebunden sein. Die cycloaliphatischen Reste können mit einem Halogen-Rest und/oder einer Hydroxyl-Gruppe, vorzugsweise mit Fluor und/oder Chlor substituiert sein.

Die cycloallphatischen Reste können bevorzugt ausgewählt werden aus der Gruppe bestehend aus gegebenenfalls wenigstens einfach substituiertem Cyclopropyl, 2-Mathytcyclopropyl, Cyclopropylmethyl, Cyclobutyl, cyclopentyl, Cyclopentylmethyl, Cyclohexyl, Cycloheptyl und Cyclooctyl.

Der Ausdruck Aryl-Rest umfaßt im Sinne der vorliegenden Erfindung auch solche aromatischen Kohlenwasserstoffreste, die mit einem gesättigten oder zumindest teilweise ungesättigten Kohlenwasserstoff-Ringsystem kondensiert sind. Bevorzugt ist als Aryl-Rest ein gegebenenfalls wenigstens einfach substituierter Phenyl- oder Naphthyl-Rest.

Sofern der Aryl-Rest mehr als einen Substituenten aufweist, können diese gleich oder verschieden sein. Diese Substituenten sind ausgewählt aus der Gruppe bestehend aus $OR^6$, Halogen, vorzugsweise F und/oder Cl, $CF_3$, CN, $NO_2$, $NR^7R^8$, $C_{1-6}$-Alkyl, $C_{1-6}$-Alkoxy, $C_{3-8}$-Cycloalkoxy und unsubstituiertem oder wenigstens einfach mit $OR^6$, Halogen, vorzugsweise F und/oder Cl, $CF_3$, CN, $NO_2$, $NR^7R^8$, $C_{1-6}$-Alkyl, $C_{1-6}$-Alkoxy oder $C_{3-8}$-Cycloalkoxy substituiertem Phenyl und unsubstituiertem oder wenigstens einfach mit $OR^6$, Halogen, vorzugsweise F und/oder Cl, $CF_3$, CN, $NO_2$, $NR^7R^8$, $C_{1-6}$-Alkyl, $C_{1-6}$-Alkoxy oder $C_{3-8}$-Cycloalkoxy substituiertem Naphtyl, wobei

$R^6$ für H, einen $C_{1-10}$-Alkyl-Rest, vorzugsweise einen $C_{1-6}$-Alkyl-Rest, einen unsubstituierten Aryl- oder Heteroaryl-Rest oder für einen unsubstituierten, über eine $C_{1-3}$-Alkylen-Gruppe gebundenen Aryl- oder Heteroaryl-Rest steht, $R^7$ und $R^8$, gleich oder verschieden, für H, einen $C_{1-10}$-Alkyl-Rest, vorzugsweise einen $C_{1-6}$-Alkyl-Rest, einen unsubstituierten Aryl- oder Heteroaryl-Rest oder für einen unsubstituierten, über eine $C_{1-3}$-Alkylen-Gruppe gebundenen Aryl- oder Heteroaryl-Rest stehen,

oder die Reste $R^7$ und $R^8$ zusammen die Gruppe $-CH_2CH_2OCH_2CH_2-$, $-CH_2CH_2NR^9CH_2CH_2-$, oder $-(CH_2)_{3-6}$ bedeuten, wobei

der Rest $R^9$ für H, einen $C_{1-10}$-Alkyl-, vorzugsweise einen $C_{1-6}$-Alkyl-, einen unsubstituierten Aryl- oder Heteroaryl-Rest oder für einen über unsubstituierten, über eine $C_{1-3}$-Alkylen-Gruppe gebundenen Aryl- oder Heteroaryl-Rest steht.

Unter einem Heteroaryl-Rest werden im Sinne der vorliegenden Erfindung auch solche heteroaromatischen, vorzugsweise 5- oder 6-gliedrigen Kohlenwasserstoff-Reste verstanden, die mit einem gesättigten oder teilweise ungesättigten Kohlenwasserstoff-Ringsystem kondensiert sind. Vorzugsweise enthalten die Heteroaryl-Reste ein oder mehrere Heteroatome ausgewählt aus der Gruppe bestehend aus Stickstoff, Sauerstoff und Schwefel. Bevorzugte Heteroaryl-Reste sind ausgewählt aus der Gruppe von unsubstituiertem oder wenigstens einfach substituiertem Furan, Benzofuran, Thiophen, Benzothiophen, Pyrrol, Pyridin, Pyrimidin, Chinolin, Isochinolin, Phthalazin und Chinazolin.

Sofern der Heteroaryl-Rest mehr als einen Substituenten aufweist, können diese gleich oder verschieden sein. Diese Substituenten sind ausgewählt aus der Gruppe bestehend aus $OR^6$, Halogen, vorzugsweise F und/oder Cl, $CF_3$, CN, $NO_2$, $NR^7R^8$, $C_{1-6}$-Alkyl, $C_{1-6}$-Alkoxy, $C_{3-8}$-Cycloalkoxy, unsubstituiertem Phenyl und unsubstituiertem Naphtyl,

wobei die Reste $R^6$, $R^7$ und $R^8$ die vorstehend angegebene Bedeutung haben. Ganz besonders bevorzugt sind folgende substituierte 1-Phenethylpiperidinverbindungen und deren entsprechende physiologisch verträglichen Salze, vorzugsweise deren Hydrochloride:

2-(1-Phenethylpiperidin-4-yl)-N-phenylacetamid,

[2-(1-Phenethylpiperidin-4-yl)ethyl]phenylamin,

2-(1-Phenethylpiperidin-4-yliden)-N-phenyl-acetamid,

N-(2-Methoxyphenyl)-2-(1-phenethylpiperidin-4-yl)-acetamid.

N-(4-Methoxyphenyl)-2-(1-phenethylpiperidin-4-yl)-acetamid,

2-(1-Phenethylpiperidin-4-yl)-N-(2-trifluormethoxyphenyl)acetamid.

(4-Methoxyphenyl)-[2-(1-phenethylpiperidin-4-yl)ethyl]amin,

2-[2-(1-Phenethylpiperidin-4-yl)ethylamino]phenol,

N-(3-Methoxyphenyl-2-(1-phenethylpiperidin-4-yl)acetamid,

N-(3-Chlor-4-methoxyphenyl)-2-(1'-phenethylpiperidin-4-yl)acetamid,

N-(4-Chlor-2-fluorphernyl)-2-(1-phenethylpiperidin-4-yl)acetamid,

2-(1-Phenethylpiperidin4-yl)-N-(3-trifluomethylphenyl)acetamid,

[2-(1-Phenethylpiperidin-4-yl)ethyl]-(3-trifluoromethylphenyl)amin,

(3-Methoxyphenyl)-[2-(1-phenethylpiperidin-4-yl)ethyl]amin,

4-[2-(1-Phenethylpiperidin-4-yl)ethylamino]phenol,

(4-Chlor-2-fluorphenyl)-[2-(1-phenethylpiperidin-4-yl)ethyl]amin,

3-[2-(1-Phenethylpiperidin-4-yl)ethylamino]phenol,

N-(3-Chlor4-methoxyphenyl)-N-[2-(1-phenethylpiperidin-4-yl)ethyl]acetamid,

N-(3-Chlor-4-methoxyphenyl)-N-[2-(1-phenethylpiperidin4-yl)ethyl]propionamid,

N-(3-Chlor-4-methoxyphenyl)-N-[2-(1-phenethylpiperidin-4-yl)ethyl]benzamid,

N-[2-(9-Phenethylpiperidin-4-yl)ethyl]-N-(3-trifluormethylphenyl)acetamid,

N-[2-(1-Phenethylpiperidin-4-yl)ethyl]-N-phenylacetamid,

N-[2-(1-Phenethylpiperidin-4-yl)ethyl]-N-phenylbenzamid,

(4-Methylpyridin-2-yl)-[2-(1-phenethyl-piperidin-4-yl)-ethyl]amin und

(4,6-Dimethyl-pyridin-2-yl)-[2-(1-phenethylpiparidin-4-yliden)-ethyl]amin.

Ein weiterer Gegenstand der vorliegenden Erfindung Ist ein Verfahren zur Herstellung von substituierten 1-Phenethylpiperidinverbindungen der vorstehend angegebenen allgemeinen Formel 1. gemäß dem

(a) 1-phenethylpiperidin-4-on der Formel II

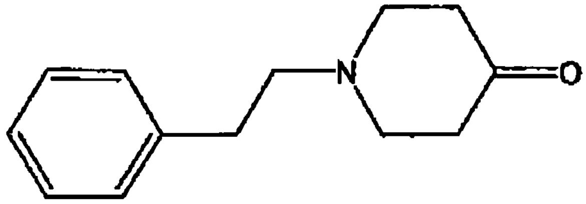

II

mit Triethylphosphonoacetat in Lösung zu (1-Phenethylpiperidin-4-yllden)-essigsäureethylester der Formel III

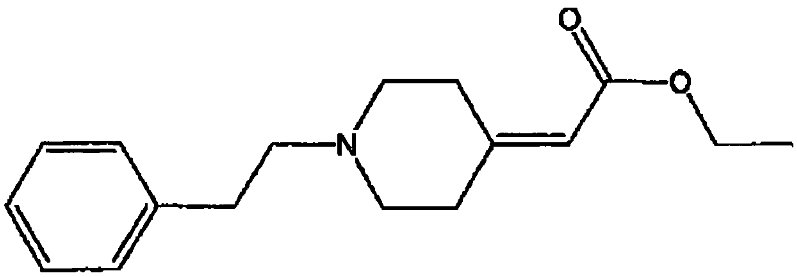

III

umgesetzt und dieser gegebenenfalls nach üblichen Methoden gereinigt und/oder gegebenenfalls nach üblichen Methoden isoliert wird,

(b) gegebenenfalls der (1-Phenethylpiperidin4-yliden)-essigsäureethylester der Formel III nach üblichen Methoden in eine Verbindung der allgemeinen Formel IV überführt,

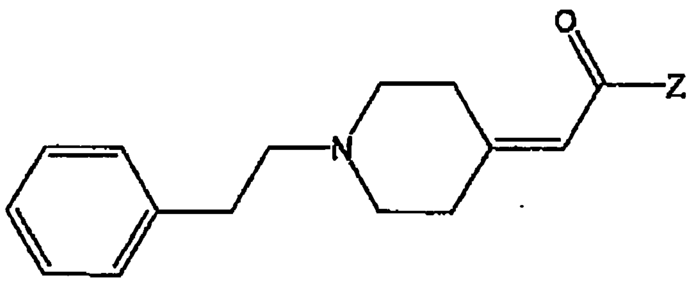

IV

worin Z für eine Gruppe steht, die das Carbonylkohlenstoffatom für die Umsetzung mit einem Amin aktiviert, die so erhaltene Verbindung der allgemeinen Formel IV gegebenenfalls nach üblichen Methoden gereinigt und/oder gegebenenfalls nach üblichen Methoden isoliert wird,

(c) gegebenenfalls wenigstens eine der Verbindungen der Formel III oder IV in Lösung zu einer entsprechenden Verbindung der allgemeinen Formel III'

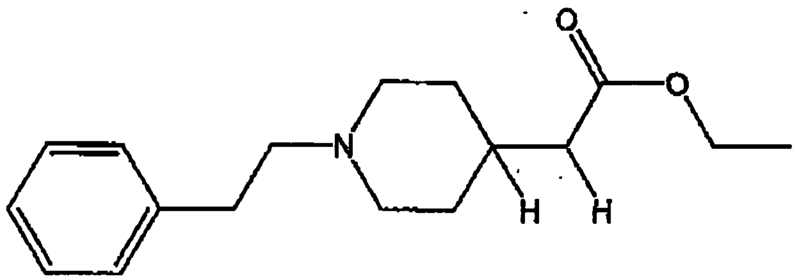

III'

oder zu einer entsprechenden Verbindung der allgemeinen Formel IV'

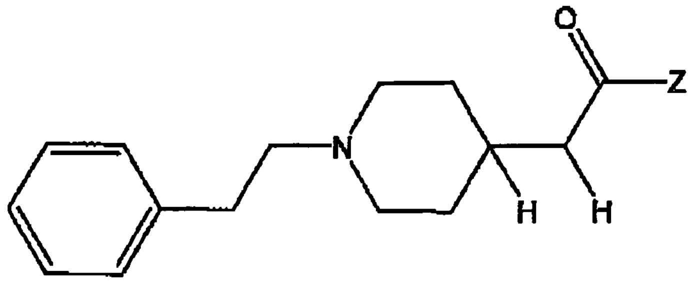

IV'

reduziert und die entsprechende Verbindung gegebenenfalls jeweils nach üblichen Methoden gereinigt und/oder gegebenenfalls jeweils nach üblichen Methoden isoliert wird,

(d) wenigstens eine Verbindung der Formel III, III', IV und IV' in Lösung mit einem primären oder sekundären Amin der allgemeinen Formel V,

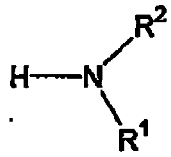

V

worin $R^1$ und $R^2$ die Bedeutung gemäß der oben angegebenen allgemeinen Formel I haben, zu wenigstens einer Verbindung der allgemeinen Formel Id

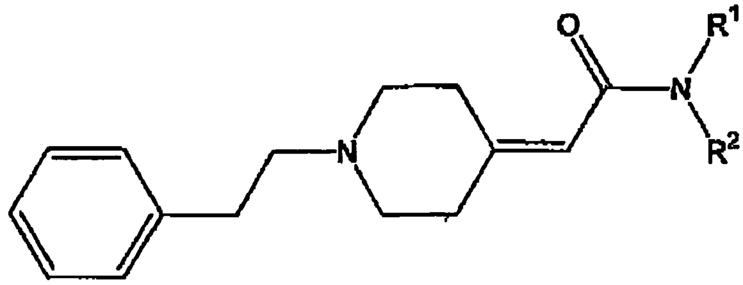

Id

und/oder wenigstens einer Verbindung der allgemeinen Formel Id'

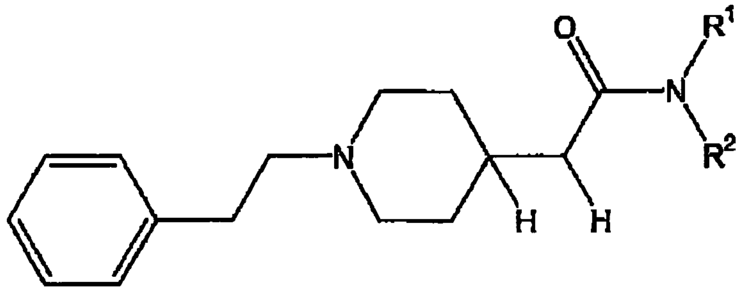

Id'

umsetzt und diese gegebenenfalls jeweils nach üblichen Methoden gereinigt und/oder gegebenenfalls jeweils nach üblichen Methoden isoliert wird,

(e) gegebenenfalls wenigstens eine der Verbindungen der allgemeinen Formel Id und/oder Id' durch Reduktion in Lösung in wenigstens eine Verbindung der allgemeinen Formel Ie

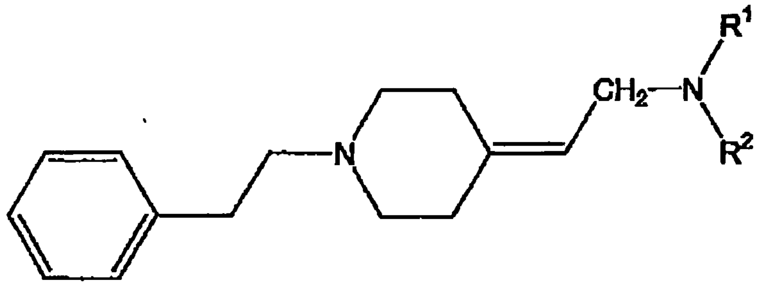

Ie

und/oder wenigstens eine Verbindung der allgemeinen Formel Ie'

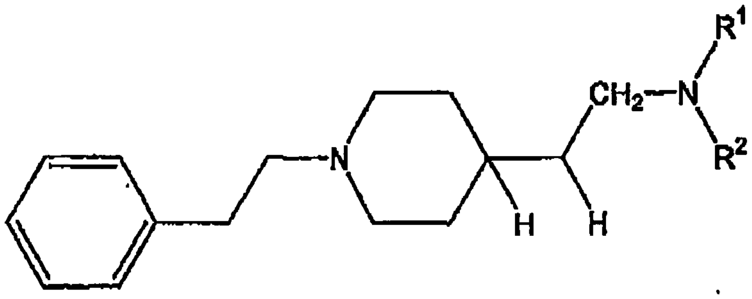

Ie'

überführt, worin $R^1$ und $R^2$ jeweils die oben angegebene Bedeutung haben, und diese gegebenenfalls jeweils nach üblichen Methoden gereinigt und/oder gegebenfalls Jeweils nach üblichen Methoden isoliert wird,

(f) gegebenenfalls wenigstens eine Verbindung der allgemeinen Formel Ie und/oder Ie', worin der Rest $R^2$ für H steht, nach üblichen, dem Fachmann bekannten Methoden In wenigstens eine Verbindung der allgemeinen Formel Ie und/oder Ie' überführt, worin der Rest $R^2$ für $COR^5$, $SO_2R^5$, einen gegebenenfalls wenigstens einfach substituierten, gesättigten, verzweigten oder unverzweigten aliphatischen $C_{1-10}$-Rest, einen gegebenenfalls wenigstens einfach substituierten, zumindest einfach ungesättigten, verzweigten oder unverzweigten aliphatischen $C_{2-10}$-Rest, einen gegebenenfalls wenigstens einfach substituierten, gesättigten oder zumindest einfach ungesättigten cycloaliphatischen $C_{3-8}$-Rest, einen gegebenenfalls wenigstens einfach substituierten Aryl- oder Hateroaryl-Rest oder für einen über eine $C_{1-3}$-AlkylenGruppe gebundenen, gegebenenfalls wenigstens einfach substituierten Aryl- oder Heteroaryl-Rest steht, wobei der Rest $R^5$ die oben angegebene Bedeutung hat und diese gegebenenfalls nach üblichen Methoden gereinigt und/oder gegebenenfalls nach üblichen Methoden isoliert wird.

Die Jeweils einzusetzenden Ausgangsverbindungen sowie die benötigten Reagenzien sind allgemein am Markt käuflich erhältlich oder können nach üblichen, dem Fachmann bekannten Methoden hergestellt werden.

Die zum Einsatz kommenden Lösungsmittel und Umsetzungsbedingungen entsprechen den für diese Typen von Reaktionen üblichen Lösungsmitteln und Umsetzungsbedingungen. Diese sind dem Fachmann beispielsweise aus A.P. Gray et al., J. Org. Chem., 26, 1961, Seiten 3368-3373, P.C. Jain et al., Indian J. Chem. 10,1972, Seiten 455-460, T. Weida et al, J. Med. Chem. 39, 1996, Seiten 380-387, H. Sugimoto et al, J: Med. Chem. 33, 1990, Seiten 1880-1887, P. Bernard et al., J. Comp. Aided Mol. Desig. 13, 1999, Seiten 365-371 und der Jeweils darin zitierten Literatur bekannt. Die entsprechenden Literaturbeschreibungen werden hiermit als Referenz eingeführt und gelten als Teil der Offenbarung.

Die Überführung des (1-Phenethylpiperidin-4-yliden)-assigsäureethylesters der Formel III in eine Verbindung der allgemeinen Formel IV, worin das Carbonylkohlenstoffatom für die Umsetzung mit einem Amin aktiviert Ist, kann nach üblichen, dem Fachmann bekannten Methoden erfolgen, wie beispielsweise in M. Bodansky, "The Peptides", Band 1. 1979, Seiten 105-196 beschrieben. Die entsprechende Literaturbeschreibung wird hiermit als Referenz eingeführt und gilt als Teil der Offenbarung.

In einer bevorzugten Ausführungsform des erfindungsgemäßen Verfahrens steht die Gruppe Z für OH, Cl oder Succinimid.

Die Reduktion der Verbindungen der Formel III bzw. IV zu den Verbindungen der Formel III' bzw. IV' kann nach üblichen, dem Fachmann bekannten Methoden erfolgen. In einer bevorzugten Ausführungsform des erfindungsgemäßen Verfahrens erfolgt die Reduktion mit Wasserstoff in Gegenwart eines Übsrgangsmetall-Katalysators, vorzugsweise in Gegenwart von Palladiumstaub, in einem geeigneten Lösungsmittel. Die Reduktion kann bei verschiedenen Wasserstoffdrücken, vorzugsweise unter einem Wasserstoffdruck von 1 bis 200 bar, vorzugsweise 1 bis 5 bar durchgeführt werden.

Die Umsetzung der Verbindungen der allgemeinen Formel III, III', IV und IV' mit primären oder sekundären Aminen der allgemeinen Formel V kann nach üblichen, dem Fachmann bekannten Methoden erfolgen. Bevorzugt erfolgt die Umsetzung mit einem primären oder sekundären Amin der allgemeinen Formel V in Gegenwart von n-Butyllithium.

Die Reduktion der Verbindung der allgemeinen Formel Id oder Id' zu Verbindungen der allgemeinen Formel Ie oder Ie' kann nach üblichen, dem Fachmann bekannten Methoden erfolgen. In einer bevorzugten Ausführungsform des erfindungsgemäßen Verfahrens erfolgt die Reduktion mit Lithiumaluminumhydrid in organischer Lösung oder mit Aluminiumhydrid (Alan), das in situ aus Lithiumaluminiumhydrid und Aluminiumchlorid gebildet wird.

Die erfindungsgemäßen substituierten 1-Phenethylpiperidinverbindungen der allgemeinen Formel I können nach dem erfindungsgemäßen Verfahren sowohl In Form Ihrer freien Base als auch in Form eines Salzes Isoliert werden. Die freie Base der jeweiligen erfindungsgemäßen Verbindung der allgemeinen Formel I kann vorzugsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, In das entsprechende physiologisch verträgliche Salz Übergeführt werden.

Die Überführung der freien Base der jeweiligen erfindungsgemäßen Verbindung der allgemeinen Formel I in das entsprechende Hydrochlorid kann ebenfalls bevorzugt auch durch Versetzen der in einem geeigneten organischen Lösungsmittel, wie z.B. Butan-2-on (Methylethylketon), gelösten erfindungsgemäßen Verbindung der allgemeinen Formel I als freie Base mit Trimethylsilylchlorid (TMSCI) erhalten wenden.

Die freie Base der jeweiligen erfindungsgemäßen Verbindung der allgemeinen Formel I kann weiterhin bevorzugt auch mit der freien Säure oder einem Salz eines Zuckerersatzstoffes, wie z.B. Saccharin. Cyclamat oder Acesulfam. In das entsprechende physiologisch verträgliche Salz übergeführt werden.

Sofern die erfindungsgemäßen substituierten 1-Phenethytpiperidinverbindungen der allgemeinen Formel I einen Phenol-Rest aufweisen, können diese durch Etherspaltung nach üblichen, dem Fachmann bekannten Methoden aus dem korrespondierenden Methylether hergestellt werden. Vorzugsweise erfolgt die Etherspaltung mit Protonen- oder Lewis-Säuren oder mit Diisobutylaluminiumhydrid.
Ebenfalls bevorzugt kann die Spaltung von Methylestern mit Aluminiumhydrid (Alan) erfolgen, das vorzugsweise in situ aus Lithiumaluminiumhydrid und Aluminiumchlorid gebildet wird.

Sofern die erfindungsgemäßen substituierten 1-Phenethylpiperidinverbindungen der allgemeinen Formel I nach dem erfindungsgemäßen Herstellungsverfahren in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 1-Phenethylpiperidinverbindungen der allgemeinen Formel I sind toxikologisch unbedenklich und eignen sich daher als pharmazeutische Wirkstoffe in Arznelmitteln.

Ein weiterer Gegenstand der vorliegenden Erfindung sind daher Arzneimittel, die wenigstens eine erfindungsgemäße substituierte 1-Phenethylpiperidinverbindung der allgemeinen Formel I sowie gegebenenfalls physiologisch verträgliche Hilfsstoffe enthalten.

Sofern die erfindungsgemäßen substituierten 1-Phenethylpiperidinverbindungen der allgemeinen Formel I oder deren entsprechende physiologisch verträgliche Salze chiral sind, können sie in Form ihrer Racemate, ihrer reinen Enantiomeren, ihrer reinen Diastereomeren oder in Form eines Gemisches aus wenigstens zwei der vorstehend genannten Stereoisomeren in dem erfindungsgemäßen Arzneimittel vorliegen. Ebenso können die erfindungsgemäßen substituierten 1-Phenethylpiperidin-verbindungen der allgemeinen Formel I auch in Form von Mischungen ihrer Enantiomeren oder Diastereomeren in dem Arzneimittel vorliegen. Diese Mischungen können die jeweiligen Stereoisomeren in jedem beliebigen Mischungsverhältnis aufweisen.

Vorzugsweise eignen sich die erfindungsgemäßen Arzneimittel zur Bekämpfung von Schmerzen oder zur Behandlung von Migräne, Diarrhoe, Harninkontinenz, Pruritus, inflammatorischen Reaktionen, allergischen Reaktionen. Alkohol- und/oder Drogen- und/oder Madikamentanabhängigkeit, Alkohol- und/oder Drogen- und/oder Metlikamentenmißbrauch, Entzündungen oder zur Lokalanästhesie.

Die Verwendung wenigstens einer substituierten 1-Phonethylpiperidinverbindung der allgemeinen Formel I zur Herstellung eines Arzneimittels zur Bekämpfung von Schmerzen, zur Behandlung von Migräne, Diarrhoe, Haminkontinenz, Pruritus, Inflammatorischen Reaktionen, allergischen Reaktionen, Alkohol- und/oder Drogen- und/oder Medikamentenabhängigkeit, Alkohol- und/oder Drogen- und/oder Medikamentenmißbrauch, Entzündungen oder zur Lokalanästhesie ist ebenfalls Gegenstand der vorliegenden Erfindung.

Die erfindungsgemäßen Arzneimittel können als flüssige, halbfeste oder feste Arzneiformen, beispielsweise in Form von Injektionslösungen, Tröpfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden,

Neben wenigstens einer erfindungsgemäßen substituierten 1-Phenethylpiperidinverbindung der allgemeinen Formel 1 enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmittel, Verdünnungsmittel, oberflächenaktiven Stoffen, Farbstoffen, Konservierungsstoffen, Sprengmittel, Gleitmittel, Schmiermittel, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel I auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der Jeweiligen erfindungsgemäßen substituierten 1-Phenethylpiperidinverbindung der allgemeinen Formel I kann variieren und ist beispielsweise abhängig vom Gewicht oder dem Alter des Patienten sowie von der Applikationsart, der indikation und dem Schweregrad der Erkrankung. Üblicherweise werden 0,005 bis 500 mg/kg, vorzugsweise 0,05 bis 5 mg/kg Körpergewicht des Patienten wenigstens einer erfindungsgemäßen substituierten 1-Phenethylpiperidinverbindung der allgemeinen Formel I appliziert.

**Pharmakologische Untersuchungen:**

1.) Analgesieprüfung im Writhing-Test an der Maus

Die Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Substanzdosis erhielten 10 Minuten nach intravenöser Gabe der getesteten Verbindungen 0,3 ml/Maus einer 0,02 %igen wäßrigen Lösung von Phenylchinon (Phenylbenzochinon, Fa, Sigma, Deisenhofen; Herstellung der Lösung unter Zusatz von 5 % Ethanol und Aufbewahrung im Wasserbad bei 45 °C) Intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhielten.

Die Verbindungen wurden in der Standarddosis von 10 mg/kg getestet Die Hemmung der Writhingreaktionen durch eine Substanz wurde nach folgender Formel berechnet:

$$\%\ \text{Hemmung} = 100 - \left[ \frac{\text{Writhingreaktion behan. Tiere}}{\text{Writhingreaktion Kontrolle}} \times 100 \right]$$

2.) Analgesieprüfung im Tail-Flick-Test an der Maus

Die Mäuse wurden Jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet

Die Lösungen der erfindungsgemäßen Verbindung der allgemeinen Formel I sowie die Vergleichslösungen

wurden dann intravenös appliziert. Die Schmerzmessung wurde jeweils 10,20,40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei Ist die Zelt $T_0$ die Latenzzeit vor der Applikation, die Zeit $T_1$ die Latenzzeit nach der Applikation der Wirkstoffkombination und die Zelt $T_2$ die maximale Expositionsdauer (12 Sekunden).

Im folgenden wird die Erfindung anhand von Beispielen erläutert. Diese Erläuterungen sind lediglich beispielhaft und schränken den allgemeinen Erfindungsgedanken nicht ein.

**Beispiele:**

Die Ausbeuten der erfindungsgemäßen Beispielverbindungen wurden nicht optimiert.

Alle Temperaturen sind unkorrigiert.

Als stationäre Phase für die Säulenchromatographie wurde Kieselgel 60 (0,040-0,063 mm) der Firma E. Merck, Darmstadt eingesetzt.

Die dünnschicht-chromatographischen Untersuchungen wurden mit HPTLC-Fertigplatten, Kieselgel 60 F 254 der Firma E. Merck, Darmstadt durchgeführt.

Die Mischungsverhältnisse von Laufmitteln für chromatographische Untersuchungen sind in Volumen/Volumen angegeben.

**(1-Phenethylpiperidin-4-yliden)essigsäureethylester (Ester 1)**

50,0 g (246 mmol) 1-Phenathylpiperidin-4-on wurden in einem Gemisch aus 200 ml Natronlauge (32 Massen-%.) und 300 ml Toluol bei Raumtemperatur gelöst. Unter Eisbadkühlung wurden 110 g (491 mmol) Triethytphosphonoacetat zugetropft, das Eisbad entfernt und das so erhaltene Reaktionsgemisch weitere 30 Minuten nachgerührt. Anschließend wurde das Reaktionsgemisch für 1,5 Stunden zum Rückfluß erhitzt.
Die organische Phase wurde abgetrennt, mit circa 100 ml Wasser gewaschen, über Natriumsulfat getrocknet, filtriert und Im Vakuum bei 500 bis 20 mbar vollständig eingeengt. Das so erhaltene Rohprodukt (60 g) wurde säutenchromaiographisch mit Ether als Laufmittel (Säulendimension: Länge 50 cm. Durchmesser 8 cm) gereinigt. Es wurden 52,8 g (1-Phenethylpiperidin-4-ylidenesssigsäureethylester erhalten, entsprechend 79 % der theoretisch berechneten Ausbeute.

**(1-Phenethylpiperidin-4-yliden)essigsäureethylester (Ester 2)**

50,0 g (182 mmol) (1-Phenethylpiperidin-4-yliden)essigsäureethylester (Ester 1) wurden in 480 ml Essigsäureethylester gelöst und nach Zugabe von 0,1 g Palladium bei einem Wasserstoffdruck von 2 bar hydriert, bis die Wasserstoffaufnahme beendet war. Die so erhaltene Reaktionsmischung wurde filtriert und im Vakuum vollständig eingeengt. Es wurden 49.0 g (1-Phenethylpiperidin-4-yliden)essigsäureethylester erhalten, entsprechend 97 % der theoretisch berechneten Ausbeute.

**Allgemeine Arbeitsvorschrift 1**

1,1 Moläquivalente des jeweiligen primären Amins wurden in Tetrahydrofuran (circa 2 ml pro mmol Amin) gelöst, unter Elsbadkühlung 2,2 Moläquivalente n-Butylllthiumlösung (1,6 mol/l in Hexan) zugetropft und eine Stunde nachgerührt. Anschließend wurde die Reaktionslösung mit Hilfe eines Trockenelsbades abgekühlt und eine Lösung das jeweiligen Esters (1 Moläquivalent) in Tetrahydrofuran (circa 0,5 ml pro mmol Ester) zugetropft. Es wurde eine Stunde unter Trockeneiskühlung nachgerührt und über Nacht aufgewärmt.
Nach Zugabe von halbgesättigter Ammoniumchloridlösung (circa 2,5 ml pro mmol Ester) wurde mehrfach mit Ether, Ethylacetat oder Dichlormethan extrahiert, die so erhaltenen Extrakte vereinigt, über Natriumsulfat getrocknet, filtriert und im Vakuum bei einem Druck von 500 bis 20 mbar vollständig eingeengt.

Zur Aufreinigung wurde das so erhaltene Rohprodukt, gegebenenfalls nach Waschen mit Hexen (circa 8 ml pro g Rohprodukt), in 2-Butanon (8,5 ml pro g Rohprodukt) gelöst, gegebenenfalls trockenes Methanol zugesetzt, um das Rohprodukt vollständig zu lösen, 0,5 Moläquivalente Wasser und 1,1 Moläquivalente Chlortrimethylsilan zugegeben und über Nacht gerührt. Das so erhaltene Hydrochlorid wurde abfiltriert und Im Hochvakuum getrocknet.

Die nach der allgemeinen Arbeitsvorschrift 1 hergestellten beispielgemäßen substituierten 1-Phenethylpiperidinverbindungen, der jeweils eingesetzte Ether, das zur Extraktion eingesetzte Lösungsmittel, die Art der Aufreinigung sowie die Ausbeute in % der theoretisch bestimmten Ausbeute sind in der nachfolgenden Tabelle 1 angegeben:

**Tabelle 1:**

| beispiel -Nr. | Verbindung | eingesetzter Ester | Extraktion | Aufreinigung | | Ausbeute in % |
|---|---|---|---|---|---|---|
| | | | | Hexan-Wäsche | Methanol-Zusatz | als Hydrochlorid |
| 1 | 2-(1-Phenethyl-piperidin-4-yl)-N-phenylacetamid | 2 | Ethylecetat | - | X | 67 |
| 3 | 2-(1-Phenethylpiperidin-4-yliden)-N-phenylacetamid | 1 | Diethylether | - | - | 77 |
| 4 | N-(2-Methoxyphenyl)-2-(1-phenethylpiperidin-4-yl)-acetamid | 2 | Ethylacetat | X | - | 73 |
| 5 | N-(4-Methoxyphenyl)-2-(1-phenethylpiperidin-4-yl)-acetamid | 2 | Dichlormethan | - | X | 76 |
| 6 | 2-(1-Phenethylpiperidin-4-yl)-N-(2-trifluormethoxyphenyl) acetamid | 2 | Ethylacetat | X | - | 63 |
| 9 | N-(3-Methoxyphenyl-2-(1-phenethylpiperidin-4-yl)acetamid | 2 | Diethylether | - | - | 69 |
| 10 | N-(3-Chlor-4-methoxyphenyl)-2-(1-phenethylpiperidin-4-yl)acetamid | 2 | Dichlormethan | - | - | 69 |
| 11 | N-(4-Chlor-2-fluorphenyl)-2-(1-phenethylpiperidin-4-yl)acetamid | 2 | Ethylacetat | X | - | 22 |
| 13 | [2-(1-phenethylpiperidin-4-yl)ethyl]-(3-trifluormethylphenyl) amin | 2 | Diethyl ether | - | - | 94 |

**Allgemeine Arbeitsvorschrift 2:**

Drei Moläquivalente Lithiumaluminiumhydrid (2,3 mol/l in Tetrahydrofuran) wurden in Tetrahydrofuran (circa 1,3 ml pro mmol Lithiumaluminiumhydrid) mit einem Moläquivalent Aluminiumchlorid umgesetzt, eine Stunde nachgerührt und anschließend ein Moläquivalent des jeweiligen Amids, gelöst in Tetrahydrofuran (circa 2 ml pro mmol Amid) zugegeben. Es wurde über Nacht bei 20 bis 25 °C gerührt. Zur Aufarbeitung wurde der Ansatz durch Zugabe von Kallumhydroxidlösung (3 mol/l) basisch gestellt und mehrfach mit Ether extrahiert. Die vereinigten Extrakte wurden über Natriumsulfat getrocknet, filtriert, eingeengt und das entsprechende Hydrochlorid gemäß der allgemeinen Arbeitsvorschrift 1 gefällt.

Die nach der allgemeinen Arbeitsvorschrift 2 hergestellten beispielgemäßen substituierten 1-Phenethylpiperidinverbindungen sowie die Ausbeute in % der theoretisch bestimmten Ausbeute sind in der nachfolgenden Tabelle 2 angegeben:

**Tabelle 2:**

| Beispiel Nr | Verbindung | Ausbeute an Hydrochlorid in % |
|---|---|---|
| 2 | [2-(1-Phenethylpiperidin-4-yl-)ethyl]phenylamin | 73 |
| 7 | (4-Methoxyphenyl)-[2-(1-phenathylpiperidin-4-yl)ethyl]amin | 97 |
| 8[a] | 2-[2-(1-Phenethylpiperidin-4-yl)ethylamino]phenol | 29 |
| 12 | 2-(1-Phenethylpiperidin-4-yl)-N-(3-trifluormethylphenyl) acetamid | 58 |
| 14 | (3-Methoxyphenyl)-[2-(1-phenethylpiperidin-4-yl)ethyl]amin | 73 |
| 16 | (4-Chlof-2-fluorphenyl)-[2-(1-phenethylpiperidin-4-yl)ethyl]amin | 70 |
| a: Bei der Herstellung dieser beispielgemäßen Verbindung wurde gleichzeitig auch der Methylether gespalten. | | |

**Allgemeine Arbeitsvorschrift 3:**

Zu drei Moläquivalenten Diisobutylaluminiumhydrid (1,5 mol/l in Toluol) wurde unter Rühren bei einer Temperatur von 20 bis 25 °C ein Moläquivalent des entsprechenden Methylethers, gelöst in Toluol (2 ml pro mmol des Methylethers), zugetropft und anschließend über Nacht zum Rückfluß erhitzt. Nach der Abkühlung wurden bei Eisbadkühlung unter Rühren nacheinander Ethanol und Wasser (Jeweils 1 ml pro mmol Ethylether) so zugetropft, daß die Temperatur nicht über 20 °C stieg. Anschließend ließ man den Ansatz für circa 4 Stunden Im Elsbad ruhen, bevor über Filtererde abgesaugt wurde. Dann wurde mit Toluol nachgewaschen, das Filtrat eingeengt und das entsprechende Hydrochlorid gemäß der allgemeinen Arbeitsvorschrift 1 gefällt.

Die nach der allgemeinen Arbeitsvorschrift 3 hergestellten beispielgemäßen substituierten 1-Phenethytpiperidinverbindungen sowie die Ausbeute in % der theoretisch bestimmten Ausbeute sind in der nachfolgenden Tabelle 3 angegeben:

**Tabelle 3:**

| Beispiel Nr | Verbindung | Ausbeute an Hydrochlorid in % |
|---|---|---|
| 15 | 4-[2-(1-Phenethylpiperidin-4-yl)-ethylamino]phenol | 42 |
| 17 | 3-[2-(1-Phenethylpiperidin-4-yl)ethylamino]phenol | 69 |

**Allgemeine Arbeitsvorschrift 4:**

Ein Äquivalent des jewelligen Amins wurde in Dichlormethan (circa 5 ml pro mmol) gelöst, eine Spatelspitze (ca. 5 bis 20 mg) 4-Dimethylaminopyridin und 1,05 Moläquivalente Triethylamin zugegeben. Der Ansatz wurde mit einem Eis/Methanol-Bad abgekühlt, 1,05 Moläquivalente des jeweiligen Säurechlorids zugetropft und anschließend zwei Stunden unter Aufwärmen bei einer Temperatur von 20 bis 25 °C nachgerührt.

Zur Aufarbeitung wurde der Ansatz mit verdünnter Kallumhydroxidlösung (ca. 5 ml pro mmol; 2-3 mol/l basisch gestellt, kurz gerührt und dann wiederholt mit Dichlormethan extrahiert. Die vereinigten Extrakte wurden über Magnesiumsulfat getrocknet, filtriert, eingeengt und das entsprechende Hydrochlorid gemäß der allgemeinen Arbeitsvorschrift 1 gefällt.

Die nach der allgemeinen Arbeitsvorschrift 4 hergestellten beispielgemäßen substituierten 1-Phenethylpiperidinverbindungen sowie die Ausbeute in % der theoretisch bestimmten Ausbeute sind in der nachfolgenden Tabelle 4 angegeben:

**Tabelle 4:**

| Beispiel Nr | Verbindung | Ausbeute an Hydrochlorid In % |
|---|---|---|
| 18 | N-(3-Chlor-4-methoxyphenyl)-N-[2-(1-phenethylpiperidin-4-yl) ethyl]acetamid | 59 |
| 19 | N-(3-Chlor-4-methoxyphenyl)-N-[2-(1-phenethylpiperidin-4-yl) ethyl]propionamid | 43 |
| 20 | N-(3-Chlor-4-methoxyphenyl)-N-[2-(1-phenethylpiperidin-4-yl) ethyl]benzamid | 59 |
| 21 | N-[2-(1-Phenethylpiperidin-4-yl)ethyl]-N-(3-trifluormethylphenyl) acetamid | 26 |
| 22 | N-[2-(1-Phenethylpiperidin-4-yl)ethyl]-N-phenylacetamid | 73 |
| 23 | N-[2-(1-Phenethylpiperidin-4-yl)ethyl]-N-phenylpropionamid | 71 |
| 24 | N-[2-(1-Phenethylpiperidin-4-yl)ethyl]-N-phenylbenzamid | 65 |

**Pharmakologische Untersuchungen:**

1.) Analgesleprüfung im Writhing-Test an der Maus:

Die vertiefte Untersuchung auf analgetische Wirksamkeit wurde im Phenylchinon-induzierten Writhing an der Maus durchgeführt, wie obenstehend beschrieben.

Die untersuchten erfindungsgemäßen Verbindungen zeigten eine analgetische Wirkung. Die Ergebnisse ausgewählter Writhing-Untersuchungett sind in der nachfolgenden Tabelle 5 zusammengefaßt

2.) Analgesleprüfung im Tail-Flick-Test an der Maus:

Die vertiefte Untersuchung auf analgetische Wirksamkeit wurde im Tail-Flick Test an der Maus durchgeführt, wie obenstehend beschrieben.

Die untersuchten erfindungsgemäßen Verbindungen zeigten eine analgetische Wirkung. Die Ergebnisse ausgewählter Untersuchungen sind ebenfalls in der nachfolgenden Tabelle 5 zusammengefaßt.

**Tabelle 5:**

| Beispiel Nr. | Antinociceptive Wirkung prozentual zur Kontrollgruppe[b] | SCHMERZMODELL |
|---|---|---|
| | | |
| 1 | 56(0,1) | Tail-Flick |
| 2 | 100(10) | Writhing |
| 3 | 69 (10) | Writhing |
| 4 | 100 (10) | Writhing |
| 5 | 100 (10) | Writhing |
| 6 | 99 (10) | Writhing |
| 7 | 100 (10) | Writhing |
| 8 | 100(10) | Writhing |
| 9 | 56(1) | Writhing |
| 10 | 100(10) | Writhing |
| 11 | 100 (10) | Writhing |
| 12 | 21(1) | Tail-Flick |
| 13 | 31(1) | Tail-Flick |

(fortgesetzt)

| Beispiel Nr. | Antinociceptive Wirkung prozentual zur Kontrollgruppe[b] | SCHMERZMODELL |
|---|---|---|
| 14 | 100(10) | Writhing |
| 15 | 100(10) | Writhing |
| 16 | 80(10) | Writhing |
| | 31(10) | Tail-Flick |
| 17 | 100 (10) | Writhing |
| 18 | 100 (10) | Writhing |
| 19 | 100 (10) | Writhing |
| | 35 (1) | Tall-Flick |
| 20 | 88 (10) | Writhing |
| | 23 (1) | Tall-Flick |
| 21 | 100 (10) | Writhing |
| | 34 (1) | Tall-Flick |
| 22 | 24 (1) | Tall-Flick |
| 23 | 99(10) | Writhing |
| 24 | 100(10) | Writhing |
| | 24(1) | Tall-Flick |
| b: In Klammem ist jeweils die Dosierung in mg/kg bei intravenöser Applikation angegeben. | | |

Die untersuchten beispielgemäßen substituierten 1-Phenethylpiperidinverbindungen zeigen eine gute analgetische Wirksamkeit.

**Patentansprüche**

**1.** Substituierte 1-Phenethylpiperidinverbindungen der allgemeinen Formel I

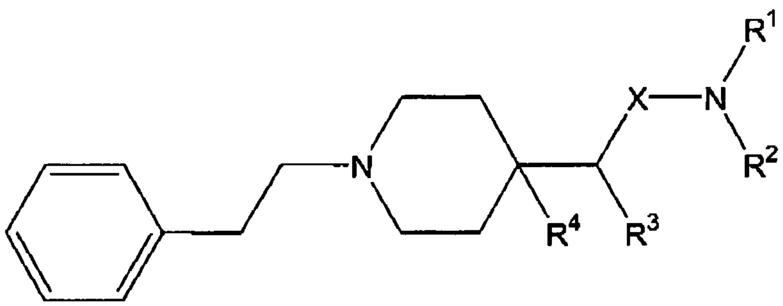

**I,**

worin

X für eine Methylen-($CH_2$)- oder Carbonyl-(C=O)-Gruppe steht,
$R^1$ für einen gegebenenfalls wenigstens einfach substituierten Aryl-Rest steht, wobei die Substituenten ausgewählt sind aus der Gruppe bestehend aus $OR^6$, Halogen, $CF_3$, CN, $NO_2$, $NR^7R^8$, $C_{1-6}$-Alkyl, $C_{1-6}$-Alkoxy, $C_{3-8}$-Cy-

cloalkoxy und unsubstituiertem oder wenigstens einfach mit $OR^6$, Halogen $CF_3$, CN, $NO_2$, $NR^7R^8$, $C_{1-6}$-Alkyl, $C_{1-6}$-Alkoxy oder $C_{3-8}$-Cycloalkoxy substituiertem Phenyl und unsubstituiertem oder wenigstens einfach mit $OR^6$, Halogen, $CF_3$, CN, $NO_2$, $NR^7R^8$, $C_{1-6}$-Alkyl, $C_{1-6}$-Alkoxy oder $C_{3-8}$-Cycloalkoxy substituiertem Naphtyl, wobei

$R^6$ für H, einen ggf. einfach oder mehrfach mit einem Halogen-Rest und/oder einer Hydroxyl-Gruppe substituierten $C_{1-10}$-Alkyl-Rest oder für einen Aryl- oder Heteroaryl-Rest ausgewählt aus der Gruppe von Phenyl, Naphthyl-Rest, Furan, Benzofuran, Thiophen, Benzothiophen, Pyrrol, Pyridin, Pyrimidin, Chinolin, Isochinolin, Phthalazin und Chinazolin steht, wobei die genannten Aryl- und Heteroaryl-Reste auch über eine $C_{1-3}$-Alkylen-Gruppe gebundenen sein können,

$R^7$ und $R^8$, gleich oder verschieden, für H, einen ggf. einfach oder mehrfach mit einem Halogen-Rest und/oder einer Hydroxyl-Gruppe substituierten $C_{1-10}$-Alkyl-Rest oder für einen Aryl- oder Heteroaryl-Rest ausgewählt aus der Gruppe von Phenyl, Naphthyl-Rest, Furan, Benzofuran, Thiophen, Benzothiophen, Pyrrol, Pyridin, Pyrimidin, Chinolin, Isochinolin, Phthalazin und Chinazolin stehen, wobei die genannten Aryl- und Heteroaryl-Reste auch über eine $C_{1-3}$-Alkylen-Gruppe gebundenen sein können,

oder die Reste $R^7$ und $R^8$ zusammen die Gruppe $-CH_2CH_2OCH_2CH_2-$, $-CH_2CH_2NR^9CH_2CH_2-$, oder $-(CH_2)_{3-6}$ bedeuten, wobei

der Rest $R^9$ für H, einen ggf. einfach oder mehrfach mit einem Halogen-Rest und/oder einer Hydroxyl-Gruppe substituierten $C_{1-10}$-Alkyl-Rest oder für einen Aryl- oder Heteroaryl-Rest ausgewählt aus der Gruppe von Phenyl, Naphthyl-Rest, Furan, Benzofuran, Thiophen, Benzothiophen, Pyrrol, Pyridin, Pyrimidin, Chinolin, Isochinolin, Phthalazin und Chinazolin steht, wobei die genannten Aryl- und Heteroaryl-Reste auch über eine $C_{1-3}$-Alkylen-Gruppe gebundenen sein können,

oder $R^1$ für einen gegebenenfalls wenigstens einfach substituierten Heteroaryl-Rest steht, wobei die Substituenten ausgewählt sind aus der Gruppe bestehend aus $OR^6$, Halogen, $CF_3$, CN, $NO_2$, $NR^7R^8$, $C_{1-6}$-Alkyl, $C_{1-6}$-Alkoxy, $C_{3-8}$-Cycloalkoxy, unsubstituiertem Phenyl und unsubstituiertem Naphtyl, und wobei die Reste $R^6$, $R^7$ und $R^8$ die vorstehend angegebene Bedeutung haben,

$R^2$ für H, $COR^5$, $SO_2R^5$, einen gegebenenfalls wenigstens einfach mit einem Halogen-Rest und/oder einer Hydroxyl-Gruppe substituierten, gesättigten, verzweigten oder unverzweigten aliphatischen $C_{1-10}$-Rest, einen gegebenenfalls wenigstens einfach mit einem Halogen-Rest und/oder einer Hydroxyl-Gruppe substituierten, zumindest einfach ungesättigten, verzweigten oder unverzweigten aliphatischen $C_{2-10}$-Rest, einen gegebenenfalls wenigstens einfach mit einem Halogen-Rest und/oder einer Hydroxyl-Gruppe substituierten, gesättigten oder zumindest einfach ungesättigten cycloaliphatischen $C_{3-8}$-Rest,

einen ggf. über eine $C_{1-3}$Alkylen-Gruppe gebundenen, gegebenenfalls wenigstens einfach substituierten Aryl-Rest, wobei die Substituenten ausgewählt sind aus der Gruppe bestehend aus Gruppe bestehend aus $OR^6$, Halogen, $CF_3$, CN, $NO_2$, $NR^7R^8$, $C_{1-6}$-Alkyl, $C_{1-6}$-Alkoxy, $C_{3-8}$-Cycloalkoxy und unsubstituiertem oder wenigstens einfach mit $OR^6$, Halogen $CF_3$, CN, $NO_2$, $NR^7R^8$, $C_{1-6}$-Alkyl, $C_{1-6}$-Alkoxy oder $C_{3-8}$-Cycloalkoxy substituiertem Phenyl und unsubstituiertem oder wenigstens einfach mit $OR^6$, Halogen, $CF_3$, CN, $NO_2$, $NR^7R^8$, $C_{1-6}$-Alkyl, $C_{1-6}$-Alkoxy oder $C_{3-8}$-Cycloalkoxy substituiertem Naphtyl, wobei die Substituenten $R^6$, $R^7$ und $R^8$ die vorstehend angegebene Bedeutung haben,

oder für einen ggf. über eine $C_{1-3}$Alkylen-Gruppe gebundenen, gegebenenfalls wenigstens einfach substituierten Heteroaryl-Rest steht, wobei die Substituenten ausgewählt sind aus der Gruppe bestehend aus $OR^6$, Halogen, $CF_3$, CN, $NO_2$, $NR^7R^8$, $C_{1-6}$-Alkyl, $C_{1-6}$-Alkoxy, $C_{3-8}$-Cycloalkoxy, unsubstituiertem Phenyl und unsubstituiertem Naphtyl, wobei die Reste $R^6$, $R^7$ und $R^8$ die vorstehend angegebene Bedeutung haben,

$R^3$ und $R^4$ jeweils für H oder zusammen für eine Bindung stehen,

$R^5$ für einen gegebenenfalls wenigstens einfach mit einem Halogen-Rest und/oder einer Hydroxyl-Gruppe substituierten, gesättigten, verzweigten oder unverzweigten aliphatischen $C_{1-10}$-Rest, einen gegebenenfalls wenigstens einfach mit einem Halogen-Rest und/oder einer Hydroxyl-Gruppe substituierten, zumindest einfach ungesättigten, verzweigten oder unverzweigten aliphatischen $C_{2-10}$-Rest, einen gegebenenfalls wenigstens einfach mit einem Halogen-Rest und/oder einer Hydroxyl-Gruppe substituierten, gesättigten oder zumindest einfach ungesättigten cycloaliphatischen $C_{3-8}$-Rest,

einen ggf. über eine $C_{1-3}$Alkylen-Gruppe gebundenen, gegebenenfalls wenigstens einfach substituierten Aryl-Rest, wobei die Substituenten ausgewählt sind aus der Gruppe bestehend aus Gruppe bestehend aus $OR^6$, Halogen, $CF_3$, CN, $NO_2$, $NR^7R^8$, $C_{1-6}$-Alkyl, $C_{1-6}$-Alkoxy, $C_{3-8}$-Cycloalkoxy und unsubstituiertem oder wenigstens einfach mit $OR^6$, Halogen $CF_3$, CN, $NO_2$, $NR^7R^8$, $C_{1-6}$-Alkyl, $C_{1-6}$-Alkoxy oder $C_{3-8}$-Cycloalkoxy substituiertem Phenyl und unsubstituiertem oder wenigstens einfach mit $OR^6$, Halogen, $CF_3$, CN, $NO_2$, $NR^7R^8$, $C_{1-6}$-Alkyl, $C_{1-6}$-Alkoxy oder $C_{3-8}$-Cycloalkoxy substituiertem Naphtyl, wobei die Substituenten $R^6$, $R^7$ und $R^8$ die vorstehend angegebene Bedeutung haben,

oder für einen ggf. über eine $C_{1-3}$Alkylen-Gruppe gebundenen, gegebenenfalls wenigstens einfach substituierten Heteroaryl-Rest steht, wobei die Substituenten ausgewählt sind aus der Gruppe bestehend aus $OR^6$, Halogen,

$CF_3$, CN, $NO_2$, $NR^7R^8$, $C_{1-6}$-Alkyl, $C_{1-6}$-Alkoxy, $C_{3-8}$-Cycloalkoxy, unsubstituiertem Phenyl und unsubstituiertem Naphtyl, wobei die Reste $R^6$, $R^7$ und $R^8$ die vorstehend angegebene Bedeutung haben,
als freie Base oder eines entsprechenden physiologisch verträglichen Salzes sowie entsprechender Racemate, Enantiomeren und Diastereomeren.

**2.** Substituierte 1-Phenethylpiperidinverbindungen gemäß Anspruch 1, **dadurch gekennzeichnet, daß** X für eine Methylen-($CH_2$)-Gruppe steht.

**3.** Substituierte 1-Phenethylpiperidinverbindungen gemäß Anspruch 1 oder 2, **dadurch gekennzeichnet, daß** $R^1$ für einen gegebenenfalls wenigstens einfach substituierten Aryl-Rest steht, wobei die möglichen Substituenten wie in Anspruch 1 definiert sind.

**4.** Substituierte 1-Phenethylpiperidinverbindungen gemäß einem der Ansprüche 1 bis 3, **dadurch gekennzeichnet, daß** $R^2$ für H, $COR^5$, $SO_2R^5$ oder für einen $C_{1-6}$-Alkyl-Rest, vorzugsweise für H oder $COR^5$ steht.

**5.** Substituierte 1-Phenethylpiperidinverbindungen gemäß einem der Ansprüche 1 bis 4, **dadurch gekennzeichnet, daß** die Reste $R^3$ und $R^4$ jeweils für H stehen.

**6.** Substituierte 1-Phenethylpiperidinverbindungen gemäß einem der Ansprüche 1 bis 5, **dadurch gekennzeichnet, daß** der Rest $R^5$ für einen $C_{1-6}$-Alkyl-Rest oder für einen unsubstituierten oder wenigstens einfach substituierten Aryl-Rest steht, wobei die möglichen Substituenten des ArylRestes wie in Anspruch 1 definiert sind.

**7.** Substituierte 1-Phenethylpiperidinverbindungen gemäß einem oder mehreren der Ansprüche 1-6:

2-(1-Phenethylpiperidin-4-yl)-N-phenylacetamid,
[2-(1-Phenethylpiperidin-4-yl-)ethyl]phenylamin,
2-(1-Phenethylpiperidin-4-yliden)-N-phenyl-acetamid,
N-(2-Methoxyphenyl)-2-(1-phenethylpiperidin-4-yl)-acetamid,
N-(4-Methoxyphenyl)-2-(1-phenethylpiperidin-4-yl)-acetamid,
2-(1-Phenethylpiperidin-4-yl)-N-(2-trifluormethoxyphenyl)acetamid,
(4-Methoxyphenyl)-[2-(1-phenethylpiperidin-4-yl)ethyl]amin,
2-[2-(1-Phenethylpiperidin-4-yl)ethylamino]phenol,
N-(3-Methoxyphenyl-2-(1-phenethylpiperidin-4-yl)acetamid,
N-(3-Chlor-4-methoxyphenyl)-2-(1-phenethylpiperidin-4-yl)acetamid,
N-(4-Chlor-2-fluorphenyl)-2-(1-phenethylpiperidin-4-yl)acetamid,
2-(1-Phenethylpiperidin-4-yl)-N-(3-trifluormethylphenyl)acetamid,
[2-(1-Phenethylpiperidin-4-yl)ethyl]-(3-trifluormethylphenyl)amin,
(3-Methoxyphenyl)-[2-(1-phenethylpiperidin-4-yl)ethyl]amin,
4-[2-(1-Phenethylpiperidin-4-yl)ethylamino]phenol,
(4-Chlor-2-fluorphenyl)-[2-(1-phenethylpiperidin-4-yl)ethyl]amin,
3-[2-(1-Phenethylpiperidin-4-yl)ethylamino]phenol,
N-(3-Chlor-4-methoxyphenyl)-N-[2-(1-phenethylpiperidin-4-yl)ethyl]acetamid,
N-(3-Chlor-4-methoxyphenyl)-N-[2-(1-phenethylpiperidin-4-yl)ethyl]propionamid,
N-(3-Chlor-4-methoxyphenyl)-N-[2-(1-phenethylpiperidin-4-yl)ethyl]benzamid,
N-[2-(1-Phenethylpiperidin-4-yl)ethyl]-N-(3-trifluormethylphenyl)acetamid,
N-[2-(1-Phenethylpiperidin-4-yl)ethyl]-N-phenylacetamid,
N-[2-(1-Phenethylpipendin-4-yl)ethyl]-N-phenylbenzamid,
(4-Methylpyridin-2-yl)-[2-(1-phenethyl-piperidin-4-yl)-ethyl]amin und
(4,6-Dimethyl-pyridin-2-yl)-[2-(1-phenethylpiperidin-4-yliden)-ethyl]amin

und deren entsprechende physiologisch verträglichen Salze, vorzugsweise deren Hydrochloride.

**8.** Verfahren zur Herstellung von substituierten 1-Phenethylpiperidinverbindungen der allgemeinen Formel I gemäß einem der Ansprüche 1 bis 7, **dadurch gekennzeichnet, daß**

(a) 1-Phenethylpiperidin-4-on der Formel II

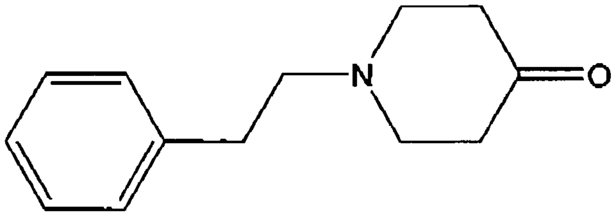

II

mit Triethylphosphonoacetat in Lösung zu (1-Phenethylpiperidin-4-yliden)-essigsäureethylester der Formel III

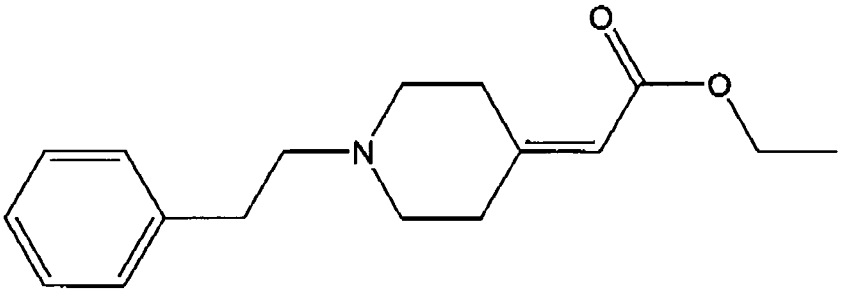

III

umgesetzt und dieser gegebenenfalls nach üblichen Methoden gereinigt und/oder gegebenenfalls nach üblichen Methoden isoliert wird,
(b) gegebenenfalls der (1-Phenethylpiperidin-4-yliden)-essigsäureethylester der Formel III nach üblichen Methoden in eine Verbindung der allgemeinen Formel IV überführt,

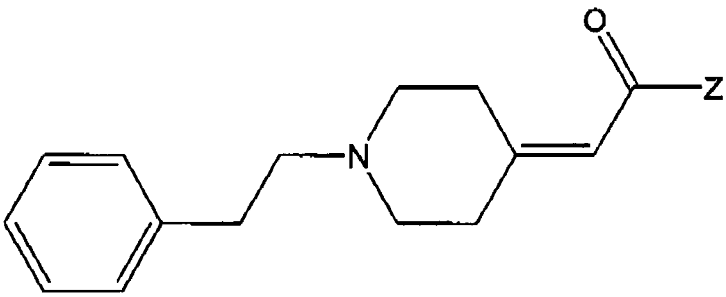

IV

worin Z für eine Gruppe steht, die das Carbonylkohlenstoffatom für die Umsetzung mit einem Amin aktiviert, die so erhaltene Verbindung der allgemeinen Formel IV gegebenenfalls nach üblichen Methoden gereinigt und/oder gegebenenfalls nach üblichen Methoden isoliert wird,
(c) gegebenenfalls wenigstens eine der Verbindungen der Formel III oder IV in Lösung zu einer entsprechenden

Verbindung der allgemeinen Formel III'

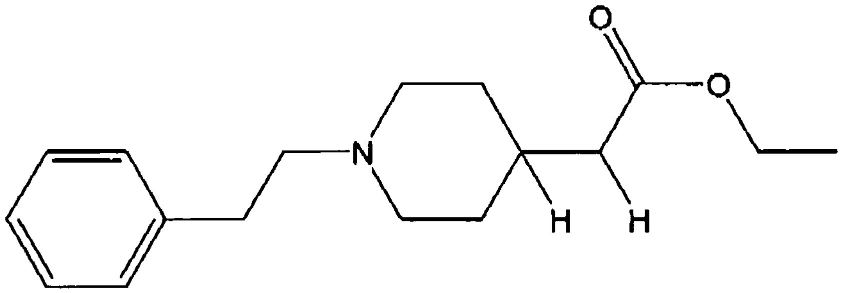

III'

oder zu einer entsprechenden Verbindung der allgemeinen Formel IV'

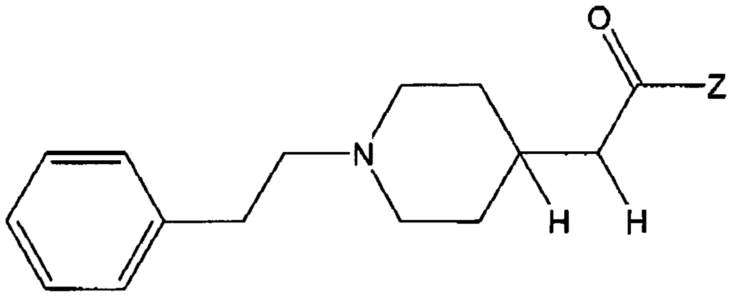

IV'

reduziert und die entsprechende Verbindung gegebenenfalls jeweils nach üblichen Methoden gereinigt und/oder gegebenenfalls jeweils nach üblichen Methoden isoliert wird,
(d) wenigstens eine Verbindung der Formel III, III', IV und IV' in Lösung mit einem primären oder sekundären Amin der allgemeinen Formel V,

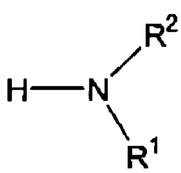

V

worin $R^1$ und $R^2$ die Bedeutung gemäß der oben angegebenen allgemeinen Formel I haben, zu wenigstens einer Verbindung der allgemeinen Formel Id

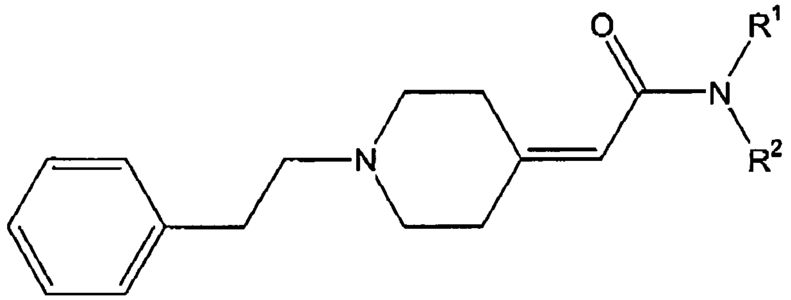

Id

und/oder wenigstens einer Verbindung der allgemeinen Formel Id'

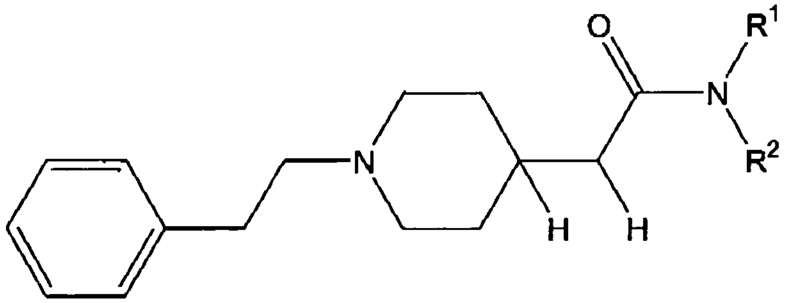

Id'

umsetzt und diese gegebenenfalls jeweils nach üblichen Methoden gereinigt und/oder gegebenenfalls jeweils nach üblichen Methoden isoliert wird,
(e) gegebenenfalls wenigstens eine der Verbindungen der allgemeinen Formel Id und/oder Id' durch Reduktion in Lösung in wenigstens eine Verbindung der allgemeinen Formel Ie

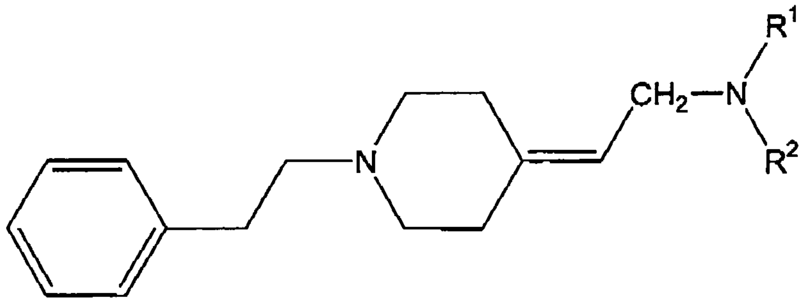

Ie

und/oder wenigstens eine Verbindung der allgemeinen Formel Ie'

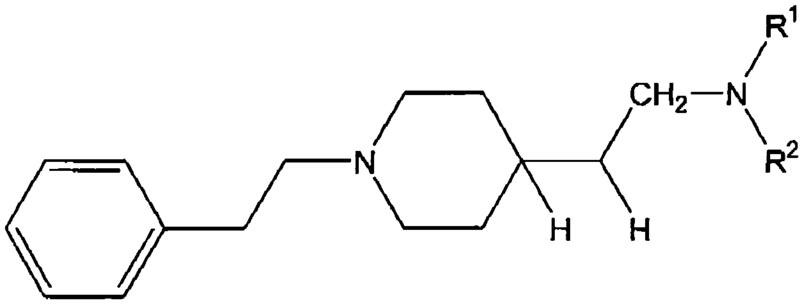

Ie'

überführt, worin $R^1$ und $R^2$ jeweils Bedeutung gemäß Anspruch 1 haben, und diese gegebenenfalls jeweils nach üblichen Methoden gereinigt und/oder gegebenfalls jeweils nach üblichen Methoden isoliert wird,
(f) gegebenenfalls wenigstens eine Verbindung der allgemeinen Formel Ie und/oder Ie', worin der Rest $R^2$ für H steht, nach üblichen, dem Fachmann bekannten Methoden in wenigstens eine Verbindung der allgemeinen Formel Ie und/oder Ie' überführt, worin der Rest $R^2$ für $COR^5$, $SO_2R^5$, einen gegebenenfalls wenigstens einfach substituierten, gesättigten, verzweigten oder unverzweigten aliphatischen $C_{1-10}$-Rest, einen gegebenenfalls wenigstens einfach substituierten, zumindest einfach ungesättigten, verzweigten oder unverzweigten aliphatischen $C_{2-10}$-Rest, einen gegebenenfalls wenigstens einfach substituierten, gesättigten oder zumindest einfach ungesättigten cycloaliphatischen $C_{3-8}$-Rest, einen gegebenenfalls wenigstens einfach substituierten Aryl- oder Heteroaryl-Rest oder für einen über eine $C_{1-3}$-Alkylen-Gruppe gebundenen, gegebenenfalls wenigstens einfach substituierten Aryl- oder Heteroaryl-Rest steht, wobei der Rest $R^5$ die oben angegebene Bedeutung hat und diese gegebenenfalls nach üblichen Methoden gereinigt und/oder gegebenenfalls nach üblichen Methoden isoliert wird.

**9.** Verfahren gemäß Anspruch 8, **dadurch gekennzeichnet, daß** Z für OH, Cl oder einen Succinimid-Rest steht.

**10.** Verfahren gemäß Anspruch 8 oder 9, **dadurch gekennzeichnet, daß** die Reduktion zu den Verbindungen der Formel III' oder IV' mit Wasserstoff in Gegenwart eines Übergangsmetall-Katalysators, vorzugsweise in Gegenwart von Palladiumstaub durchgeführt wird.

**11.** Verfahren gemäß einem der Ansprüche 8 bis 10, **dadurch gekennzeichnet, daß** die Umsetzung mit einem primären oder sekundären Amin der allgemeinen Formel V in Gegenwart von n-Butyllithium durchgeführt wird.

**12.** Verfahren gemäß einem der Ansprüche 8 bis 11, **dadurch gekennzeichnet, daß** die Reduktion zu einer Verbindung der allgemeinen Formel Ie oder Ie' mit in situ aus Lithiumaluminiumhydrid und Aluminiumtrichlorid erzeugten Aluminiumhydrid (Alan) in einem organischen Lösungsmittel erfolgt.

**13.** Arzneimittel enthaltend wenigstens eine substituierte 1-Phenethylpiperidinverbindung gemäß einem der Ansprüche 1 bis 7 und gegebenenfalls physiologisch verträgliche Hilfsstoffe.

**14.** Arzneimittel gemäß Anspruch 13 zur Bekämpfung von Schmerzen.

**15.** Arzneimittel gemäß Anspruch 13 zur Behandlung von Migräne.

**16.** Arzneimittel gemäß Anspruch 13 zur Behandlung von Diarrhoe.

**17.** Arzneimittel gemäß Anspruch 13 zur Behandlung von Harninkontinenz.

**18.** Arzneimittel gemäß Anspruch 13 zur Behandlung von Pruritus.

**19.** Arzneimittel gemäß Anspruch 13 zur Behandlung von inflammatorischen Reaktionen.

**20.** Arzneimittel gemäß Anspruch 13 zur Behandlung von allergischen Reaktionen.

**21.** Arzneimittel gemäß Anspruch 13 zur Behandlung von Alkohol- und/oder Drogen- und/oder Medikamentenmißbrauch.

**22.** Arzneimittel gemäß Anspruch 13 zur Behandlung von Alkohol- und/oder Drogen- und/oder Medikamentenabhängigkeit.

**23.** Arzneimittel gemäß Anspruch 13 zur Behandlung von Entzündungen.

**24.** Arzneimittel gemäß Anspruch 13 zur Lokalanästhesie.

**25.** Verwendung wenigstens einer substituierten 1-Phenethylpiperidinverbindung gemäß einem der Ansprüche 1 bis 7 zur Herstellung eines Arzneimittels zur Bekämpfung von Schmerz oder zur Behandlung von Migräne, Diarrhoe, Haminkontinenz, Pruritus, inflammatorischen Reaktionen, allergischen Reaktionen, Alkohol- und/oder Drogen- und/oder Medikamentenmißbrauch, Alkohol- und/oder Drogen- und/oder Medikamentenabhängigkeit oder Entzündungen oder zur Lokalanästhesie.

## Claims

**1.** Substituted 1-phenethylpiperidine compounds of the general formula I

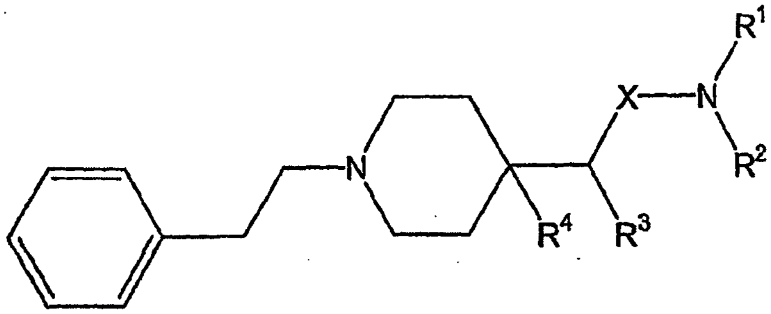

I,

in which

X denotes a methylene ($CH_2$) or carbonyl (C=O) group,
$R^1$ denotes an optionally at least mono-substituted aryl residue, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy and unsubstituted phenyl or phenyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy and unsubstituted naphthyl or naphthyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy, wherein
$R^6$ denotes H, a $C_{1-10}$ alkyl residue optionally mono- or polysubstituted with a halogen residue and/or a hydroxyl residue, or an aryl or heteroaryl residue selected from the group of phenyl, naphthyl residue, furan, benzofuran, thiophene, benzothiophene, pyrrole, pyridine, pyrimidine, quinoline, isoquinoline, phthalazine and quinazoline, wherein the stated aryl and heteroaryl residues may also be attached via a $C_{1-3}$ alkylene group,
$R^7$ and $R^8$, identical or different, denote H, a $C_{1-10}$ alkyl residue optionally mono- or polysubstituted with a halogen residue and/or a hydroxyl group or an aryl or heteroaryl residue selected from the group phenyl, naphthyl residue, furan, benzofuran, thiophene, benzothiophene, pyrrole, pyridine, pyrimidine, quinoline, isoquinoline, phthalazine and quinazoline, wherein the stated aryl and heteroaryl residues may also be attached via a $C_{1-3}$ alkylene group,

or the residues $R^7$ and $R^8$ together mean the group $-CH_2CH_2OCH_2CH_2-$, $-CH_2CH_2NR^9CH_2CH_2-$, or $-(CH_2)_{3-6}$, wherein

the residue $R^9$ denotes H, a $C_{1-10}$ alkyl optionally mono-or polysubstituted with a halogen residue and/or a hydroxyl group, or an aryl or heteroaryl residue selected from the group phenyl, naphthyl residue, furan, benzofuran, thiophene, benzothiophene, pyrrole, pyridine, pyrimidine, quinoline, isoquinoline, phthalazine and quinazoline, wherein the stated aryl and heteroaryl residues may also be attached via a $C_{1-3}$ alkylene group,

or $R^1$ denotes an optionally at least monosubstituted heteroaryl residue, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy, unsubstituted phenyl and unsubstituted naphthyl, and wherein the residues $R^6$, $R^7$ and $R^8$ have the above-stated meaning,

$R^2$ denotes H, $COR^5$, $SO_2R^5$, a saturated, branched or unbranched aliphatic $C_{1-10}$ residue optionally at least mono-substituted with a halogen residue and/or a hydroxyl group, an at least mono-unsaturated, branched or unbranched aliphatic $C_{2-10}$ residue optionally at least mono-substituted with a halogen residue and/or a hydroxyl group, a saturated or at least mono-unsaturated cycloaliphatic $C_{3-8}$ residue optionally at least mono-substituted with a halogen residue and/or a hydroxyl group,

an optionally at least mono-substituted aryl residue optionally attached via a $C_{1-3}$ alkylene group, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy and unsubstituted phenyl or phenyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy and unsubstituted naphthyl or naphthyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy, wherein the residues $R^6$, $R^7$ and $R^8$ have the above-stated meaning,

or an optionally at least mono-substituted heteroaryl residue optionally attached via a $C_{1-3}$ alkylene group, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy, unsubstituted phenyl and unsubstituted naphthyl, wherein the residues $R^6$, $R^7$ and $R^8$ have the above-stated meaning,

$R^3$ and $R^4$ each separately denote H or together denote a bond,

$R^5$ denotes a saturated, branched or unbranched aliphatic $C_{1-10}$ residue optionally at least mono-substituted with a halogen residue and/or a hydroxyl group, an at least mono-unsaturated, branched or unbranched aliphatic $C_{2-10}$ residue optionally at least mono-substituted with a halogen residue and/or a hydroxyl group, a saturated or at least mono-unsaturated cycloaliphatic $C_{3-8}$ residue optionally at least mono-substituted with a halogen residue and/or a hydroxyl group,

an optionally at least mono-substituted aryl residue optionally attached via a $C_{1-3}$ alkylene group, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy and unsubstituted phenyl or phenyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy and unsubstituted naphthyl or naphthyl at least mono-substituted with $OR^6$, halogen, $CF_3$, CN, $NO_2$, $NR^7R^8$, $C_{1-6}$ alkyl, $C_{1-6}$ alkoxy or $C_{3-8}$ cycloalkoxy, wherein the residues $R^6$, $R^7$ and $R^8$ have the above-stated meaning,

or an optionally at least mono-substituted heteroaryl residue optionally attached via a $C_{1-3}$ alkylene group, wherein the substituents are selected from the group consisting of $OR^6$, halogen, $CF_3$, CN, $NO_2$, $NR^7R^8$, $C_{1-6}$ alkyl, $C_{1-6}$ alkoxy, $C_{3-8}$ cycloalkoxy, unsubstituted phenyl and unsubstituted naphthyl, wherein the residues $R^6$, $R^7$ and $R^8$ have the above-stated meaning,

as a free base or a corresponding physiologically acceptable salt and corresponding racemates, enantiomers and diastereomers.

2. Substituted 1-phenethylpiperidine compounds according to claim 1, **characterised in that** X denotes a methylene ($CH_2$) group.

3. Substituted 1-phenethylpiperidine compounds according to claim 1 or claim 2, **characterised in that** $R^1$ denotes an optionally at least mono-substituted aryl residue, wherein the possible substituents are defined as in claim 1.

4. Substituted 1-phenethylpiperidine compounds according to any one of claims 1 to 3, **characterised in that** $R^2$ denotes H, $COR^5$, $SO_2R^5$ or a $C_{1-6}$ alkyl residue, preferably H or $COR^5$.

5. Substituted 1-phenethylpiperidine compounds according to any one of claims 1 to 4, **characterised in that** the residues $R^3$ and $R^4$ each denote H.

6. Substituted 1-phenethylpiperidine compounds according to any one of claims 1 to 5, **characterised in that** the residue $R^5$ denotes a $C_{1-6}$ alkyl residue or an unsubstituted or at least mono-substituted aryl residue as defined in

claim 1.

**7.** Substituted 1-phenethylpiperidine compounds according to one or more of claims 1-6:

2-(1-phenethylpiperidin-4-yl)-N-phenylacetamide,
[2-(1-phenethylpiperidin-4-yl-)ethyl]phenylamine,
2-(1-phenethylpiperidin-4-ylidene)-N-phenyl-acetamide,
N-(2-methoxyphenyl)-2-(1-phenethylpiperidin-4-yl)-acetamide,
N-(4-methoxyphenyl)-2-(1-phenethylpiperidin-4-yl)-acetamide,
2-(1-phenethylpiperidin-4-yl)-N-(2-trifluoromethoxyphenyl)acetamide,
(4-methoxyphenyl)-[2-(1-phenethylpiperidin-4-yl)ethyl]amine,
2-[2-(1-phenethylpiperidin-4-yl)ethylamino]phenol,
N-(3-methoxyphenyl)-2-(1-phenethylpiperidin-4-yl)acetamide,
N-(3-chloro-4-methoxyphenyl)-2-(1-phenethylpiperidin-4-yl)acetamide,
N-(4-chloro-2-fluorophenyl)-2-(l-phenethylpiperidin-4-yl)acetamide,
2-(1-phenethylpiperidin-4-yl)-N-(3-trifluoromethylphenyl)acetamide,
[2-(1-phenethylpiperidin-4-yl)ethyl]-(3-trifluoromethylphenyl)amine,
(3-methoxyphenyl)-[2-(1-phenethylpiperidin-4-yl)ethyl]amine,
4-[2-(1-phenethylpiperidin-4-yl)ethylamino]phenol,
(4-chloro-2-fluorophenyl)-[2-(1-phenethylpiperidin-4-yl)ethyl]amine,
3-[2-(1-phenethylpiperidin-4-yl)ethylamino]phenol,
N-(3-chloro-4-methoxyphenyl)-N-[2-(1-phenethylpiperidin-4-yl)ethyl]acetamide,
N-(3-chloro-4-methoxyphenyl)-N-[2-(1-phenethylpiperidin-4-yl)ethyl]propionamide,
N-(3-chloro-4-methoxyphenyl)-N-[2-(1-phenethylpiperidin-4-yl)ethyl]benzamide,
N-[2-(1-phenethylpiperidin-4-yl)ethyl]-N-(3-trifluoromethylphenyl)acetamide,
N-[2-(1-phenethylpiperidin-4-yl)ethyl]-N-phenylacetamide,
N-[2-(1-phenethylpiperidin-4-yl)ethyl]-N-phenylbenzamide,
(4-methylpyridin-2-yl)-[2-(1-phenethyl-piperidin-4-yl)-ethyl]amine and
(4,6-dimethyl-pyridin-2-yl)-[2-(1-phenethylpiperidin-4-ylidene)-ethyl]amine

and the corresponding physiologically acceptable salts thereof, preferably the hydrochlorides thereof.

**8.** A method for the production of substituted 1-phenethylpiperidine compounds of the general formula I according to any one of claims 1 to 7, **characterised in that**

(a) 1-phenethylpiperidin-4-one of the formula II

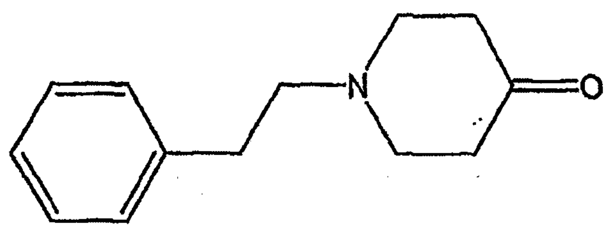

II

is reacted with triethyl phosphonoacetate in solution to yield (1-phenethylpiperidin-4-ylidene)-ethyl acetate of the formula III

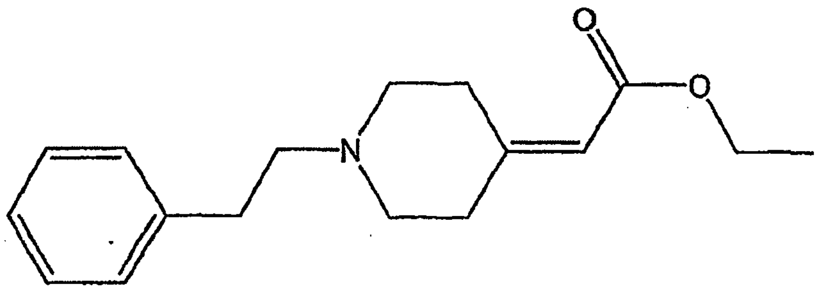

III

and this is optionally purified in accordance with conventional methods and/or optionally isolated in accordance with conventional methods,
(b) optionally the (1-phenethylpiperidin-4-ylidene)-ethyl acetate of the formula III is converted in accordance with conventional methods into a compound of the general formula IV,

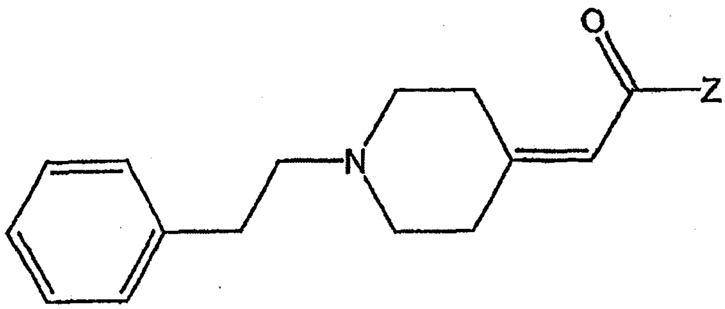

IV

in which Z denotes a group which activates the carbonyl carbon atom for reaction with an amine, the compound of the general formula IV thus obtained is optionally purified in accordance with conventional methods and/or optionally isolated in accordance with conventional methods,
(c) optionally at least one of the compounds of the formula III or IV in solution is reduced to yield a corresponding compound of the general formula III'

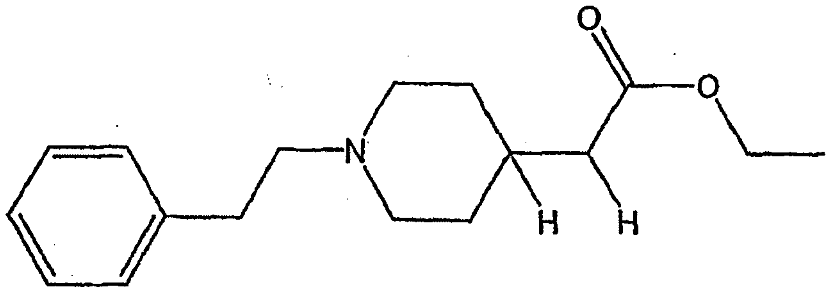

III'

or to yield a corresponding compound of the general formula IV'

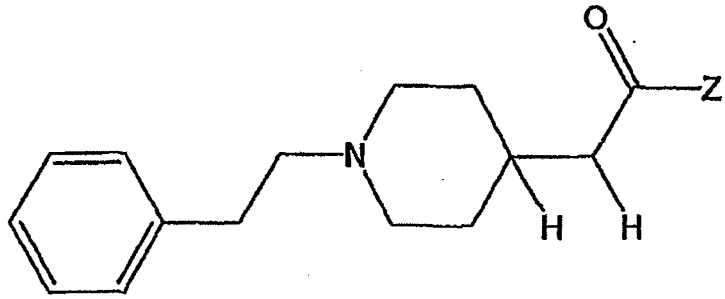

IV'

and the corresponding compound is optionally purified in each case in accordance with conventional methods and/or optionally isolated in each case in accordance with conventional methods,

(d) at least one compound of the formula III, III', IV and IV' in solution is reacted with a primary or secondary amine of the general formula V,

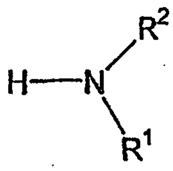

V

in which $R^1$ and $R^2$ have the meaning according to the above-stated general formula I, to yield at least one compound of the general formula Id

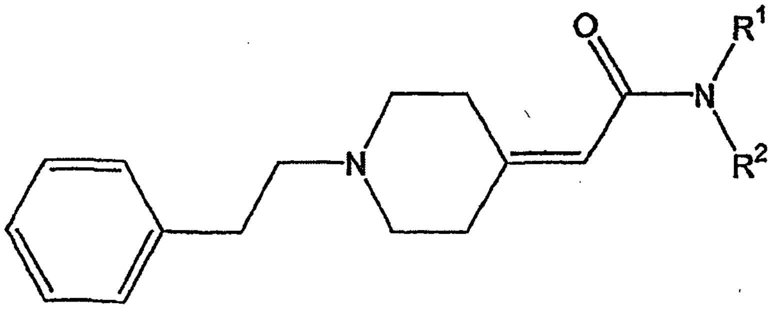

Id

and/or at least one compound of the general formula Id'

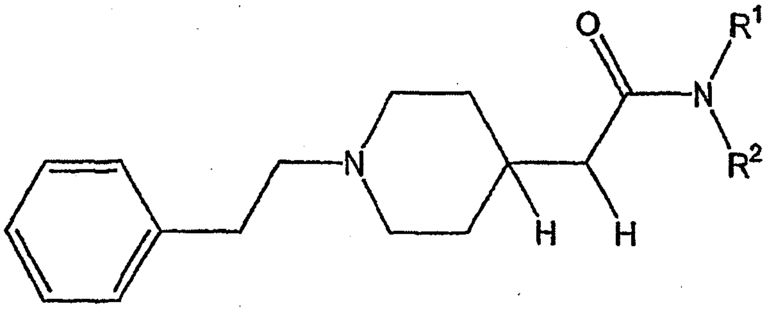

Id'

and this is optionally purified in each case in accordance with conventional methods and/or optionally isolated in each case in accordance with conventional methods,

(e) optionally at least one of the compounds of the general formula Id and/or Id' is converted by reduction in solution into at least one compound of the general formula Ie

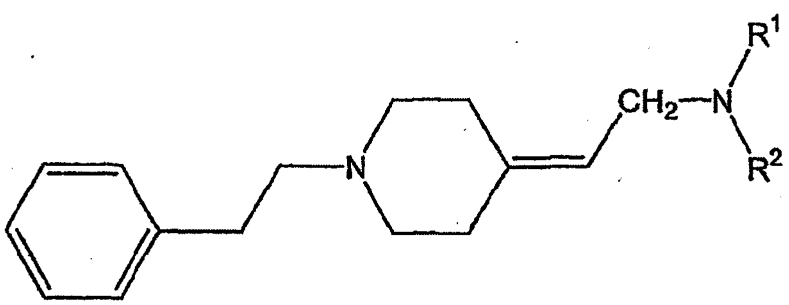

Ie

and/or at least one compound of the general formula Ie'

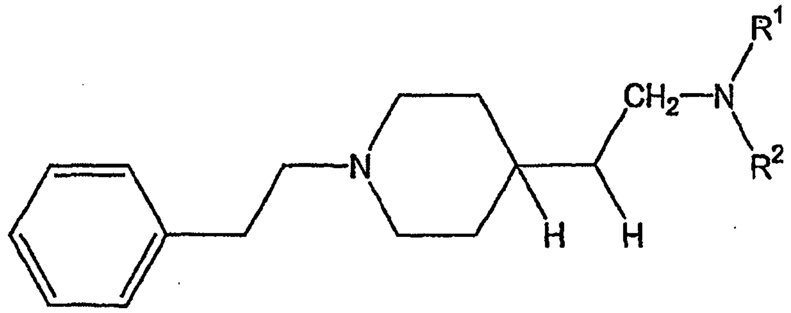

Ie'

in which $R^1$ and $R^2$ each have the meaning according to claim 1, and this is optionally purified in each case in accordance with conventional methods and/or optionally isolated in each case in accordance with conventional methods,

(f) optionally at least one compound of the general formula Ie and/or Ie', in which the residue $R^2$ denotes H, is converted in accordance with conventional methods known to the person skilled in the art into at least one compound of the general formula Ie and/or Ie', in which the residue $R^2$ denotes $COR^5$, $SO_2R^5$, an optionally at least mono-substituted, saturated, branched or unbranched aliphatic $C_{1-10}$ residue, an optionally at least mono-substituted, at least mono-unsaturated, branched or unbranched aliphatic $C_{2-10}$ residue, an optionally at least mono-substituted, saturated or at least mono-unsaturated cycloaliphatic $C_{3-8}$ residue, an optionally at least mono-substituted aryl or heteroaryl residue or an optionally at least mono-substituted aryl or heteroaryl residue attached via a $C_{1-3}$ alkylene group, wherein the residue $R^5$ has the above-stated meaning and this is optionally purified in accordance with conventional methods and/or optionally isolated in accordance with conventional methods.

**9.** A method according to claim 8, **characterised in that** Z denotes OH, Cl or a succinimide residue.

**10.** A method according to claim 8 or claim 9, **characterised in that** the reduction to yield the compounds of formula III' or IV' is performed with hydrogen in the presence of a transition metal catalyst, preferably in the presence of palladium powder.

**11.** A method according to any one of claims 8 to 10, **characterised in that** the reaction with a primary or secondary amine of the general formula V is performed in the presence of n-butyllithium.

**12.** A method according to any one of claims 8 to 11, **characterised in that** reduction to yield a compound of the general formula Ie or Ie' proceeds with aluminium hydride (alane) produced in situ from lithium aluminium hydride and aluminium trichloride in an organic solvent.

**13.** A medicament containing at least one substituted 1-phenethylpiperidine compound according to any one of claims 1 to 7 and optionally physiologically acceptable auxiliary substances.

**14.** A medicament according to claim 13 for combatting pain.

**15.** A medicament according to claim 13 for the treatment of migraine.

**16.** A medicament according to claim 13 for the treatment of diarrhoea.

**17.** A medicament according to claim 13 for the treatment of urinary incontinence.

**18.** A medicament according to claim 13 for the treatment of pruritus.

**19.** A medicament according to claim 13 for the treatment of inflammatory reactions.

**20.** A medicament according to claim 13 for the treatment of allergic reactions.

**21.** A medicament according to claim 13 for the treatment of the abuse of alcohol and/or drugs and/or medicines.

**22.** A medicament according to claim 13 for the treatment of dependency on alcohol and/or drugs and/or medicines.

**23.** A medicament according to claim 13 for the treatment of inflammation.

**24.** A medicament according to claim 13 for local anaesthesia.

**25.** Use of at least one substituted 1-phenethylpiperidine compound according to any one of claims 1 to 7 to produce a medicament for combatting of pain, for the treatment of migraine, diarrhoea, urinary incontinence, pruritus, inflammatory reactions, allergic reactions, dependency on alcohol and/or drugs and/or medicines, abuse of alcohol and/or drugs and/or medicines, inflammation or for local anaesthesia.

**Revendications**

**1.** Composés substitués de 1-phénéthylpipéridine de formule générale I

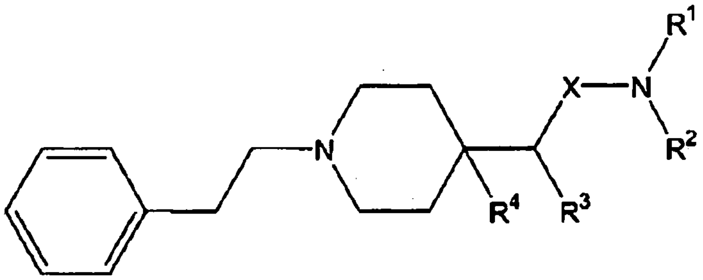

**I,**

où

X représente un groupe méthylène-($CH_2$) ou carbonyle-(C=O),
$R^1$ représente un radical aryle, le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$-cycloalcoxy et phényle non substitué ou au moins monosubstitué par $OR^6$, halogène, $CF_3$, CN, $NO_2$, $NR^7R^8$, $C_{1-6}$-alkyle, $C_{1-6}$-alcoxy ou $C_{3-8}$-cycloalcoxy et naphtyle non substitué ou au moins monosubstitué par $OR^6$, halogène, $CF_3$, CN, $NO_2$, $NR^7R^8$, $C_{1-6}$-alkyle, $C_{1-6}$-alcoxy ou $C_{3-8}$-cycloalcoxy, où
$R^6$ représente H, un radical $C_{1-10}$-alkyle le cas échéant monosubstitué ou polysubstitué par un radical halogéno et/ou un groupe hydroxyle ou un radical aryle ou hétéroaryle, choisi dans le groupe formé par phényle, un radical naphtyle, furanne, benzofuranne, thiophène, benzothiophène, pyrrole, pyridine, pyrimidine, quinoléine, isoquinoléine, phtalazine et quinazoline, les radicaux aryle et hétéroaryle mentionnés pouvant également être liés via un groupe $C_{1-3}$-alkylène,
$R^7$ et $R^8$ sont identiques ou différents et représentent H, un radical $C_{1-10}$-alkyle le cas échéant monosubstitué ou polysubstitué par un radical halogéno et/ou un groupe hydroxyle ou un radical aryle ou hétéroaryle, choisi dans le groupe formé par phényle, un radical naphtyle, furanne, benzofuranne, thiophène, benzothiophène, pyrrole, pyridine, pyrimidine, quinoléine, isoquinoléine, phtalazine et quinazoline, les radicaux aryle et hétéroaryle mentionnés pouvant également être liés via un groupe $C_{1-3}$-alkylène,

ou les radicaux $R^7$ et $R^8$ signifient ensemble le groupe $-CH_2CH_2OCH_2CH_2-$, $-CH_2CH_2NR^9CH_2CH_2-$, ou $-(CH_2)_{3-6}$, où le radical $R^9$ représente H, un radical $C_{1-10}$-alkyle le cas échéant monosubstitué ou polysubstitué par un radical

halogéno et/ou un groupe hydroxyle ou un radical aryle ou hétéroaryle, choisi dans le groupe formé par phényle, un radical naphtyle, furanne, benzofuranne, thiophène, benzothiophène, pyrrole, pyridine, pyrimidine, quinoléine, isoquinoléine, phtalazine et quinazoline, les radicaux aryle et hétéroaryle mentionnés pouvant également être liés via un groupe $C_{1-3}$-alkylène,
ou $R^1$ représente un radical hétéroaryle le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$-cycloalcoxy, phényle non substitué et naphtyle non substitué, et où les radicaux $R^6$, $R^7$ et $R^8$ ont la signification susmentionnée,
$R^2$ représente H, $COR^5$, $SO_2R^5$, un radical en $C_{1-10}$ le cas échéant au moins monosubstitué par un radical halogéno et/ou un groupe hydroxyle, saturé, ramifié ou non ramifié, aliphatique, un radical en $C_{2-10}$ le cas échéant au moins monosubstitué par un radical halogéno et/ou un groupe hydroxyle, au moins monoinsaturé, ramifié ou non ramifié, aliphatique, un radical en $C_{3-8}$ le cas échéant au moins monosubstitué par un radical halogéno et/ou un groupe hydroxyle, saturé ou au moins monoinsaturé, cycloaliphatique,
un radical aryle le cas échéant lié via un groupe $C_{1-3}$-alkylène, le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$-cycloalcoxy et phényle non substitué ou au moins monosubstitué par $OR^6$, halogène $CF_3$, CN, $NO_2$, $NR^7R^8$, $C_{1-6}$-alkyle; $C_{1-6}$-alcoxy ou $C_{3-6}$-cycloalcoxy et naphtyle non substitué ou au moins monosubstitué par $OR^6$, halogène, $CF_3$, CN, $NO_2$, $NR^7R^8$, $C_{1-6}$-alkyle, $C_{1-6}$-alcoxy ou $C_{3-8}$-cycloalcoxy, où les substituants $R^6$, $R^7$ et $R^8$ ont la signification susmentionnée,
ou un radical hétéroaryle le cas échéant lié via un groupe $C_{1-3}$-alkylène, le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$-cycloalcoxy, phényle non substitué et naphtyle non substitué, où les radicaux $R^6$, $R^7$ et $R^8$ ont la signification susmentionnée,
$R^3$ et $R^4$ représentent à chaque fois H ou, ensemble, une liaison,

$R^5$ représente un radical en $C_{1-10}$ le cas échéant au moins monosubstitué par un radical halogéno et/ou un groupe hydroxyle, saturé, ramifié ou non ramifié, aliphatique, un radical en $C_{2-10}$ le cas échéant au moins monosubstitué par un radical halogéno et/ou un groupe hydroxyle, au moins monoinsaturé, ramifié ou non ramifié, aliphatique, un radical en $C_{3-8}$ le cas échéant au moins monosubstitué par un radical halogéno et/ou un groupe hydroxyle, saturé ou au moins monoinsaturé, cycloaliphatique,

un radical aryle le cas échéant lié via un groupe $C_{1-3}$-alkylène, le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$-cycloalcoxy et phényle non substitué ou au moins monosubstitué par $OR^6$, halogène, $CF_3$, CN, $NO_2$, $NR^7R^8$, $C_{1-6}$-alkyle, $C_{1-6}$-alcoxy ou $C_{3-8}$-cycloalcoxy et naphtyle non substitué ou au moins monosubstitué par $OR^6$, halogène, $CF_3$, CN, $NO_2$, $NR^7R^8$, $C_{1-6}$-alkyle, $C_{1-6}$-alcoxy ou $C_{3-8}$-cycloalcoxy, où les substituants $R^6$, $R^7$ et $R^8$ ont la signification susmentionnée,
ou un radical hétéroaryle le cas échéant lié via un groupe $C_{1-3}$-alkylène, le cas échéant au moins monosubstitué, les substituants étant choisis dans le groupe constitué par $OR^6$, halogène, $CF_3$, CN, $NO_2$, $NR^7R^8$, $C_{1-6}$-alkyle, $C_{1-6}$-alcoxy, $C_{3-8}$cycloalcoxy, phényle non substitué et naphtyle non substitué, où les radicaux $R^6$, $R^7$ et $R^8$ ont la signification susmentionnée,
sous forme de base libre ou d'un sel physiologiquement acceptable correspondant ainsi que les racémates, énantiomères et diastéréo-isomères correspondants.

2. Composés substitués de 1-phénéthylpipéridine selon la revendication 1, **caractérisés en ce que** X représente un groupe méthylène-($CH_2$).

3. Composés substitués de 1-phénéthylpipéridine selon la revendication 1 ou 2, **caractérisés en ce que** $R^1$ représente un radical aryle le cas échéant au moins monosubstitué, les substituants possibles étant définis comme dans la revendication 1.

4. Composés substitués de 1-phénéthylpipéridine selon l'une quelconque des revendications 1 à 3, **caractérisés en ce que** $R^2$ représente H, $COR^5$, $SO_2R^5$ ou un radical $C_{1-6}$-alkyle, de préférence H ou $COR^5$.

5. Composés substitués de 1-phénéthylpipéridine selon l'une quelconque des revendications 1 à 4, **caractérisés en ce que** les radicaux $R^3$ et $R^4$ représentent chacun H.

6. Composés substitués de 1-phénéthylpipéridine selon l'une quelconque des revendications 1 à 5, **caractérisés en ce que** le radical $R^5$ représente un radical $C_{1-6}$-alkyle ou un radical aryle non substitué ou au moins monosubstitué,

les substituants possibles du radical aryle étant définis comme dans la revendication 1.

7. Composés substitués de 1-phénéthylpipéridine selon l'une ou plusieurs des revendications 1-6 :

2-(1-phénéthylpipéridin-4-yl)-N-phénylacétamide,
[2-(1-phénéthylpipéridin-4-yl-)éthyl]phénylamine,
2-(1-phénéthylpipéridin-4-ylidène)-N-phénylacétamide,
N-(2-méthoxyphényl)-2-(1-phénéthylpipéridin-4-yl)-acétamide,
N-(4-méthoxyphényl)-2-(1-phénéthylpipéridin-4-yl)-acétamide,
2-(1-phénéthylpipéridin-4-yl)-N-(2-trifluorométhoxyphényl)acétamide,
(4-méthoxyphényl)-[2-(1-phénéthylpipéridin-4-yl)éthyl]amine,
2-[2-(1-phénéthylpipéridin-4-yl)éthylamino]phénol,
N-(3-méthoxyphényl-2-(1-phénéthylpipéridin-4-yl)acétamide,
N-(3-chloro-4-méthoxyphényl)-2-(1-phénéthylpipéridin-4-yl)acétamide,
N-(4-chloro-2-fluorophényl)-2-(1-phénéthylpipéridin-4-yl)acétamide,
2-(1-phénéthylpipéridin-4-yl)-N-(3-trifluorométhylphényl)acétamide,
[2-(1-phénéthylpipéridin-4-yl)éthyl]-(3-trifluorométhylphényl)amine,
(3-méthoxyphényl)-[2-(1-phénéthylpipéridin-4-yl)éthyl]amine,
4-[2-(1-phénéthylpipéridin-4-yl)éthylamino]phénol,
(4-chloro-2-fluorophényl)-[2-(1-phénéthylpipéridin-4-yl)éthyl]amine,
3-[2-(1-phénéthylpipéridin-4-yl)éthylamino]phénol,
N-(3-chloro-4-méthoxyphényl)-N-[2-(1-phénéthylpipéridin-4-yl)éthyl]acétamide,
N-(3-chloro-4-méthoxyphényl)-N-[2-(1-phénéthylpipéridin-4-yl)éthyl]propionamide,
N-(3-chloro-4-méthoxyphényl)-N-[2-(1-phénéthylpipéridin-4-yl)éthyl]benzamide,
N-[2-(1-phénéthylpipéridin-4-yl)éthyl]-N-(3-trifluorométhylphényl)acétamide,
N-[2-(1-phénéthylpipéridin-4-yl)éthyl]-N-phénylacétamide,
N-[2-(1-phénéthylpipéridin-4-yl)éthyl]-N-phénylbenzamide,
(4-méthylpyridin-2-yl)-[2-(1-phénéthylpipéridin-4-yl)-éthyl]amine et
(4,6-diméthylpyridin-2-yl)-[2-(1-phénéthylpipéridin-4-ylidène)-éthyl]amine

et leurs sels physiologiquement acceptables correspondants, de préférence leurs chlorhydrates.

8. Procédé pour la préparation de composés substitués de 1-phénéthylpipéridine de formule générale I selon l'une quelconque des revendications 1 à 7, **caractérisé**

(a) **en ce qu'**on transforme de la 1-phénéthylpipéridin-4-one de formule II

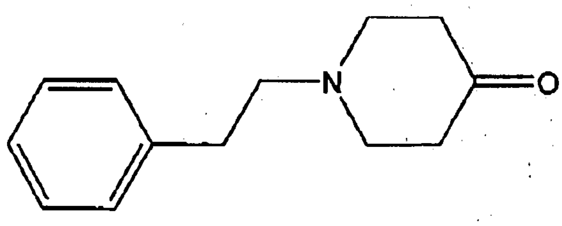

II

avec du triéthylphosphonoacétate en solution en ester éthylique de l'acide (1-phénéthylpipéridin-4-ylidène)-acétique de formule III

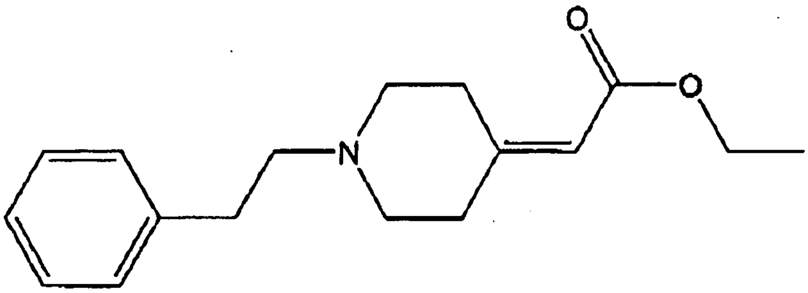

III

et celui-ci est le cas échéant purifié selon des procédés usuels et/ou le cas échéant isolé selon des procédés usuels,

(b) **en ce qu'**on transforme le cas échéant l'ester éthylique de l'acide (1-phénéthylpipéridin-4-ylidène)-acétique de formule III selon des procédés usuels en un composé de formule générale IV,

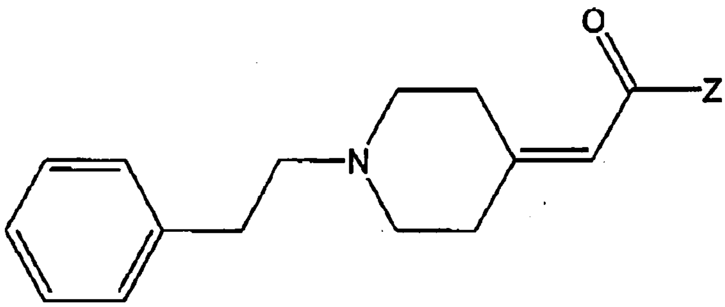

IV

dans laquelle Z représente un groupe qui active l'atome de carbone de la fonction carbonyle pour la transformation avec une amine, le composé ainsi obtenu de formule générale IV est le cas échéant purifié selon des procédés connus et/ou le cas échéant isolé selon des procédés usuels,

(c) **en ce qu'**on réduit le cas échéant au moins un des composés de formule III ou IV en solution en un composé correspondant de formule générale III'

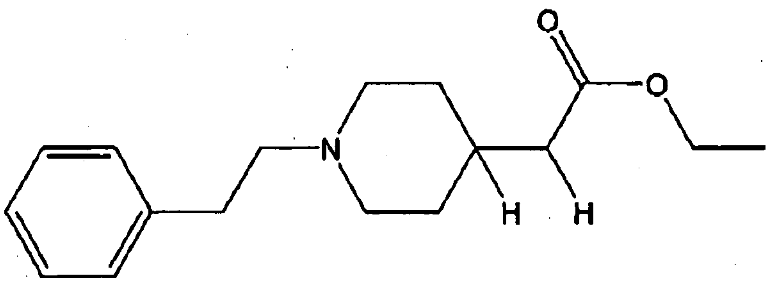

III'

ou en un composé correspondant de formule générale IV'

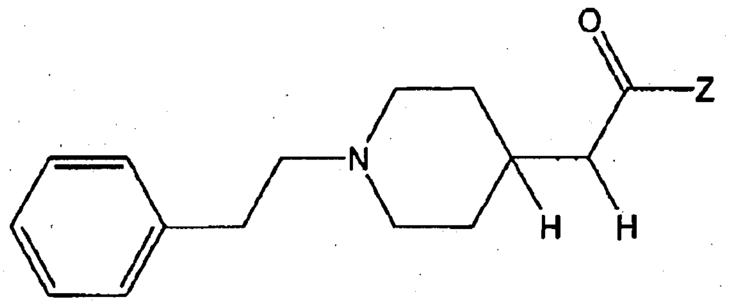

IV'

et le composé correspondant est le cas échéant purifié à chaque fois selon des procédés usuels et/ou le cas échéant isolé à chaque fois selon des procédés usuels,
(d) **en ce qu'**on transforme au moins un composé de formule III, III', IV et IV' en solution avec une amine primaire ou secondaire de formule générale V,

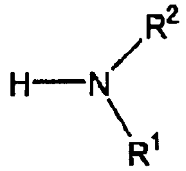

V

où $R^1$ et $R^2$ ont la signification selon la formule générale I indiquée ci-dessus en au moins un composé de formule générale Id

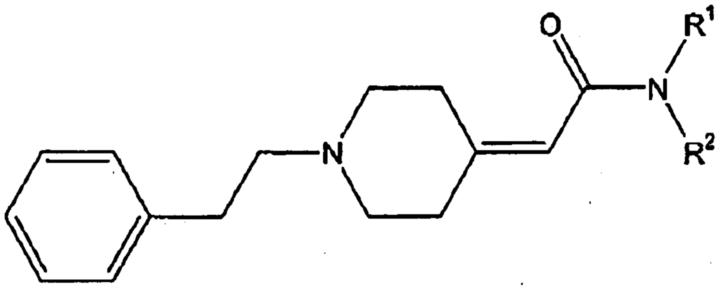

Id

et/ou au moins un composé de formule générale Id'

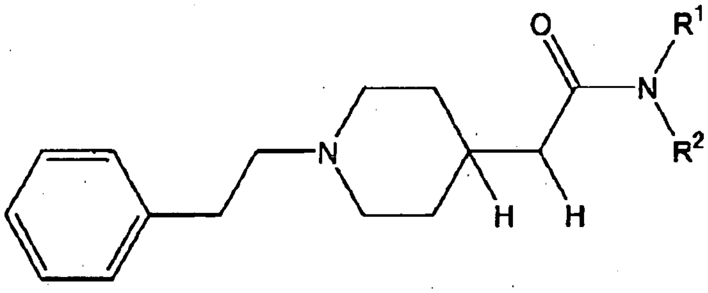

**Id'**

et celui-ci est le cas échéant purifié à chaque fois selon des procédés usuels et/ou le cas échéant isolé à chaque fois selon des procédés usuels,

(e) **en ce qu'**on transforme le cas échéant au moins un des composés de formule générale Id et/ou Id' par réduction en solution en au moins un composé de formule générale Ie

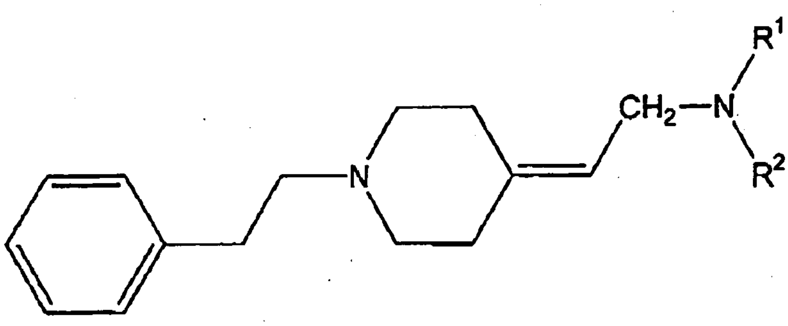

**Ie**

et/ou au moins un composé de formule générale Ie'

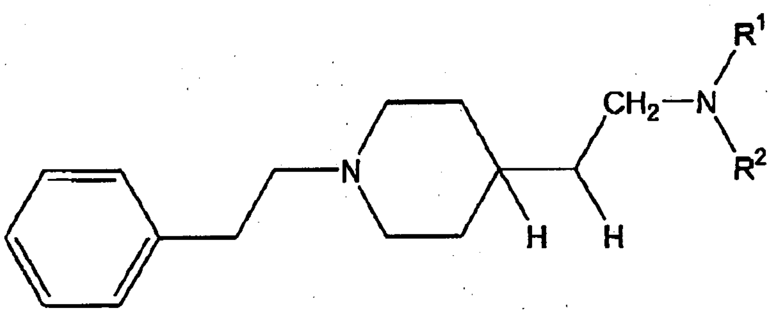

**Ie'**

$R^1$ et $R^2$ ayant à chaque fois la signification selon la revendication 1 et celui-ci est le cas échéant purifié à chaque fois selon des procédés usuels et/ou le cas échéant isolé à chaque fois selon des procédés usuels,
(f) **en ce qu'**on transforme le cas échéant au moins un composé de formule générale Ie et/ou Ie', où le radical $R^2$ représente H, selon des procédés usuels, connus par l'homme de métier, en au moins un composé de formule générale Ie et/ou Ie', où le radical $R^2$ représente $COR^5$, $SO_2R^5$, un radical en $C_{1-10}$ le cas échéant au moins monosubstitué, saturé, ramifié ou non ramifié, aliphatique, un radical en $C_{2-10}$ le cas échéant au moins monosubstitué, au moins monoinsaturé, ramifié ou non ramifié, aliphatique, un radical en $C_{3-8}$ le cas échéant au moins monosubstitué, saturé ou au moins monoinsaturé, cycloaliphatique, un radical aryle

ou hétéroaryle le cas échéant au moins monosubstitué ou un radical aryle ou hétéroaryle lié via un groupe $C_{1-3}$-alkylène, le cas échéant au moins monosubstitué, le radical $R^5$ ayant la signification susmentionnée et ce composé étant le cas échéant purifié selon des procédés usuels et/ou le cas échéant isolé selon des procédés usuels.

**9.** Procédé selon la revendication 8, **caractérisé en ce que** Z représente OH, C1 ou un radical succinimide.

**10.** Procédé selon la revendication 8 ou 9, **caractérisé en ce que** la réduction en composés de formule III' ou IV' est réalisée avec de l'hydrogène en présence d'un catalyseur de métal de transition, de préférence en présence de poussière de palladium.

**11.** Procédé selon l'une quelconque des revendications 8 à 10, **caractérisé en ce que** la transformation avec une amine primaire ou secondaire de formule générale V est réalisée en présence de n-butyllithium.

**12.** Procédé selon l'une quelconque des revendications 8 à 11, **caractérisé en ce que** la réduction en un composé de formule générale Ie ou Ie' a lieu avec de l'hydrure d'aluminium (Alan) produit in situ à partir d'hydrure de lithium et d'aluminium et de trichlorure d'aluminium dans un solvant organique.

**13.** Médicament contenant au moins un composé substitué de 1-phénéthylpipéridine selon l'une quelconque des revendications 1 à 7 et le cas échéant des adjuvants physiologiquement acceptables.

**14.** Médicament selon la revendication 13 destiné à lutter contre des douleurs.

**15.** Médicament selon la revendication 13 destiné au traitement de migraines.

**16.** Médicament selon la revendication 13 destiné au traitement de la diarrhée.

**17.** Médicament selon la revendication 13 destiné au traitement de l'incontinence urinaire.

**18.** Médicament selon la revendication 13 destiné au traitement du prurit.

**19.** Médicament selon la revendication 13 destiné au traitement de réactions inflammatoires.

**20.** Médicament selon la revendication 13 destiné au traitement de réactions allergiques.

**21.** Médicament selon la revendication 13 destiné au traitement de l'abus d'alcool et/ou de drogues et/ou de médicaments.

**22.** Médicament selon la revendication 13 destiné au traitement de la dépendance à l'alcool et/ou aux drogues et/ou aux médicaments.

**23.** Médicament selon la revendication 13 destiné au traitement d'inflammations.

**24.** Médicament selon la revendication 13 destiné à l'anesthésie locale.

**25.** Utilisation d'au moins un composé substitué de 1-phénéthylpipéridine selon l'une quelconque des revendications 1 à 7 pour la préparation d'un médicament destiné à lutter contre la douleur ou au traitement de migraines, de diarrhée, de l'incontinence urinaire, du prurit, de réactions inflammatoires, de réactions allergiques, de l'abus d'alcool et/ou de drogues et/ou de médicaments, de la dépendance à l'alcool et/ou aux drogues et/ou aux médicaments ou d'inflammations ou à l'anesthésie locale.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4248877 A


### In der Beschreibung aufgeführte Nicht-Patentliteratur


- **HUEGI B.S.** *J. Med. Chem,* 1983, vol. 26, 42-50
- **A.P. GRAY et al.** *J. Org. Chem.,* 1961, vol. 26, 3368-3373
- **P.C. JAIN et al.** *Indian J. Chem.,* 1972, vol. 10, 455-460
- **T. WEIDA et al.** *J. Med. Chem.,* 1996, vol. 39, 380-387
- **H. SUGIMOTO et al.** *J: Med. Chem.,* 1990, vol. 33, 1880-1887
- **P. BERNARD et al.** *J. Comp. Aided Mol. Desig.,* 1999, vol. 13, 365-371
- **M. BODANSKY.** *The Peptides,* 1979, vol. 1, 105-196
- Remington's Pharmaceutical Sciences. Mack Publishing Company, 1985, vol. 17
- **I.C. HENDERSHOT ; J. FORSAITH.** *J. Pharmacol. Exp. Ther.,* 1959, vol. 125, 237-240